# EUROPEAN PATENT APPLICATION

(11) **EP 4 299 075 A1**
(43) Date of publication of application: **03.01.2024**
(21) Application number: 22759551.9
(22) Date of filing: 21.02.2022
(51) Int. Cl.: A61K 45/06, A61K 31/167, A61K 31/195, A61K 35/12, A61K 39/395, A61P 31/00, A61P 35/00, A61P 43/00, C07K 16/28

(54) **COMBINATION THERAPY WITH PD-1 SIGNAL INHIBITOR**

(30) Priority: 26.02.2021 JP 2021031041
(71) Applicant: Kyoto University, Kyoto-shi, Kyoto 606-8501 (JP)
(72) Inventor: HONJO, Tasuku, Kyoto-shi, Kyoto 606-8501 (JP); WAKU, Yuka, Kyoto-shi, Kyoto 606-8501 (JP); CHAMOTO, Kenji, Kyoto-shi, Kyoto 606-8501 (JP)
(74) Representative: J A Kemp LLP
(86) International application number: PCT/JP2022/006843
(87) International publication number: WO 2022/181515

(57) **Abstract**

The present invention provides a novel combination therapy with PD-1 blockade therapy. A pharmaceutical composition which comprises a substance capable of enhancing T cell receptor (TCR) signaling and is administered before, after or simultaneously with the administration of a PD-1 signaling inhibitor. A drug which enhances PD-1 signaling inhibitory activity, comprising a CD45 inhibitor and/or a cell. A TCR signaling enhancer comprising a CD45 inhibitor and/or a cell.

## Description

### Technical Field

The results of recent clinical trials have revealed that the PD-1 blockade (signaling inhibition) therapy is more effective than conventional therapies in a broad spectrum of cancers¹⁻⁴ (Non-Patent Documents Nos. 1-4). The response rate of anti-PD-1 antibody therapy in terminal cancer patients was 20-30%, showing a dramatic improvement compared to conventional anti-cancer drug therapies. However, about one half of the patients were non-responsive, which raises a problem⁵⁻⁶ (Non-Patent Documents Nos. 5-6). Therefore, development of a combination therapy with PD-1 blockade therapy effective for those non-responsive patients is urgently needed.

### Prior Art Literature

### Non-Patent Documents

Non-Patent Document No. 1: S. L. Topalian et al., Safety, activity, and immune correlates of anti-PD-1 antibody in cancer. N Engl J Med 366, 2443-2454 (2012)
Non-Patent Document No. 2: A. Ribas et al., Association of Pembrolizumab With Tumor Response and Survival Among Patients With Advanced Melanoma. JAMA 315, 1600-1609 (2016)
Non-Patent Document No. 3: M. Reck et al., Pembrolizumab versus Chemotherapy for PD-L1-Positive Non-Small-Cell Lung Cancer. N Engl J Med 375, 1823-1833 (2016)
Non-Patent Document No. 4: P. S. Chowdhury, K. Chamoto, T. Honjo, Combination therapy strategies for improving PD-1 blockade efficacy: a new era in cancer immunotherapy. J Intern Med 283, 110-120 (2018)
Non-Patent Document No. 5: W. Zou, J. D. Wolchok, L. Chen, PD-L1 (B7-H1) and PD-1 pathway blockade for cancer therapy: Mechanisms, response biomarkers, and combinations. Sci Transl Med 8, 328rv324 (2016)
Non-Patent Document No. 6: A. L. Shergold, R. Millar, R. J. B. Nibbs, Understanding and overcoming the resistance of cancer to PD-1/PD-L1 blockade. Pharmacol Res 145, 104258 (2019)

### Summary of the Invention

### Problem for Solution by the Invention

It is an object of the present invention to provide a novel combination therapy with PD-1 blockade therapy.

### Means to Solve the Problem

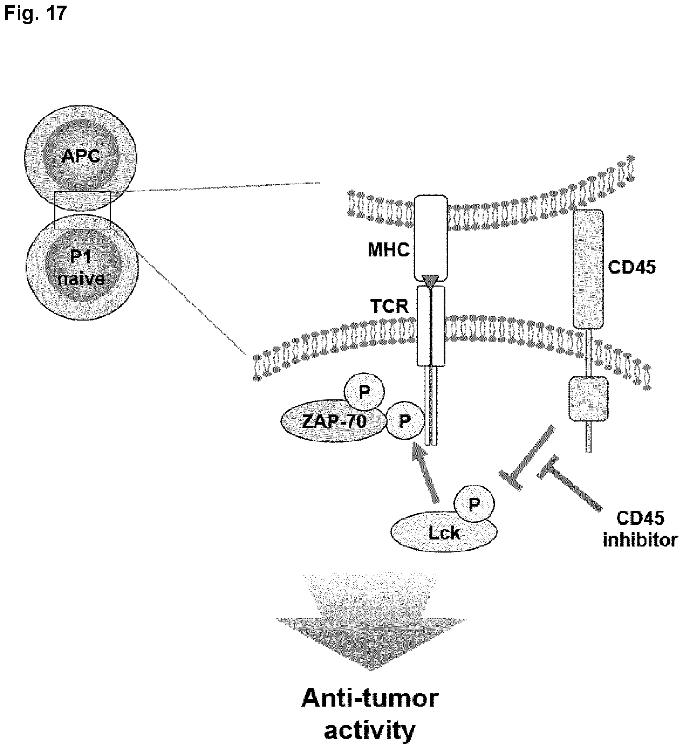

The effect of PD-1 signaling pathway blockade is mainly caused through activation of T cells (P. S. Chowdhury, K. Chamoto, T. Honjo, Combination therapy strategies for improving PD-1 blockade efficacy: a new era in cancer immunotherapy. J Intern Med 283, 110-120 (2018); K. Chamoto et al., Mitochondrial activation chemicals synergize with surface receptor PD-1 blockade for T cell-dependent antitumor activity. Proc Natl Acad Sci U S A 114, E761-E770 (2017)). This activation of T cells is promoted by T cell receptor (TCR) signaling cascades. Phosphorylation and dephosphorylation of TCR signaling molecules affect formation of signaling complexes and TCR signal transduction. CD45, which is a transmembrane-type phosphatase, plays a major role in the modulation of TCR signaling by regulating the phosphorylation level of lymphocyte protein kinase (Lck) and its downstream factor tyrosine (J. Rossy, D. J. Williamson, K. Gaus, How does the kinase Lck phosphorylate the T cell receptor? Spatial organization as a regulatory mechanism. Front Immunol 3, 167 (2012).) (R. J. Brownlie, R. Zamoyska, T cell receptor signaling networks: branched, diversified and bounded. Nat Rev Immunol 13, 257-269 (2013)). High expression of CD45 inhibits TCR signal transduction through dephosphorylation of ZAP-70, PLCg, etc. in young naive CD8⁺ T cells (J. H. Cho et al., CD45-mediated control of TCR tuning in naive and memory CD8(+) T cells. Nat Commun 7, 13373 (2016)). Therefore, it is believed that by enhancing TCR signal transduction via inhibition of CD45, T cell activation that has been augmented by PD-1 blockade would be further reinforced, resulting in enhancement of antitumor effect (Fig. 17).

The present inventors have found that antitumor effect is synergistically improved when PD-1 blockade is used in combination with an inhibitor of CD45 (phosphatase). This effect of tumor suppression was strongly exhibited, in particular, against aged mice showing resistance to PD-1 blockade or against resistant cancers. From these results, it is considered that CD45 inhibitors, when used in combination with PD-1 signaling inhibitors, support antitumor immunity, retain an effect of synergistically inhibiting cancer proliferation, and are even effective on those cancers which are non-responsive to PD-1 blockade therapy conducted alone. Similar effects were also observed when cell (xenogeneic or allogeneic) injection was conducted. Enhancement of TCR signal transduction caused by cell injection was also confirmed.

A summary of the present invention is as described below.
(1) A pharmaceutical composition which comprises a substance capable of enhancing T cell receptor (TCR) signaling and is administered before, after or simultaneously with the administration of a PD-1 signaling inhibitor.
(2) The pharmaceutical composition of (1) above, wherein the substance capable of enhancing TCR signaling is a CD45 inhibitor and/or a cell.
(3) The pharmaceutical composition of (2) above, wherein the CD45 inhibitor is at least one compound selected from the group consisting of 2-(4-acetylanilino)-3-chloronaphthoquinone, N-(9,10-dioxo-9,10-dihydrophenanthren-2-yl)-2,2-dimethylpropionamide, and analogs thereof.
(4) The pharmaceutical composition of (2) above, wherein the cell is a xenogeneic cell, an allogeneic cell or a combination thereof.
(5) The pharmaceutical composition of any one of (1) to (4) above, wherein the PD-1 signaling inhibitor is an antibody.
(6) The pharmaceutical composition of (5) above, wherein the antibody is at least one antibody selected from the group consisting of anti-PD-1 antibody, anti-PD-L1 antibody and anti-PD-L2 antibody.
(7) The pharmaceutical composition of any one of (1) to (6) above, which is used as an anti-cancer agent, an anti-infective agent or a combination thereof.
(8) A drug which enhances PD-1 signaling inhibitory activity, comprising a CD45 inhibitor and/or a cell.
(9) A TCR signaling enhancer comprising a CD45 inhibitor and/or a cell.
(10) A method of preventing and/or treating cancer, infection or a combination thereof, comprising administering to a subject a pharmaceutically effective amount of a substance capable of enhancing T cell receptor (TCR) signaling before, after or simultaneously with the administration of a PD-1 signaling inhibitor.
(11) A method of enhancing PD-1 signaling inhibitory activity, comprising administering to a subject a pharmaceutically effective amount of a substance capable of enhancing T cell receptor (TCR) signaling before, after or simultaneously with the administration of a PD-1 signaling inhibitor.
(12) A method of enhancing TCR signaling, comprising administering to a subject a pharmaceutically effective amount of a CD45 inhibitor and/or a cell.
(13) A substance capable of enhancing T cell receptor (TCR) signaling for use in prevention and/or treatment of cancer, infection or a combination thereof, said substance being administered before, after or simultaneously with the administration of a PD-1 signaling inhibitor.

### Effect of the Invention

Antitumor effect is synergistically improved if substances capable of enhancing TCR signaling are used in combination with PD-1 signaling inhibitors.

The present specification encompasses the contents disclosed in the specification and/or drawings of Japanese Patent Application No. 2021-31041 based on which the present application claims priority.

### Brief Description of the Drawings

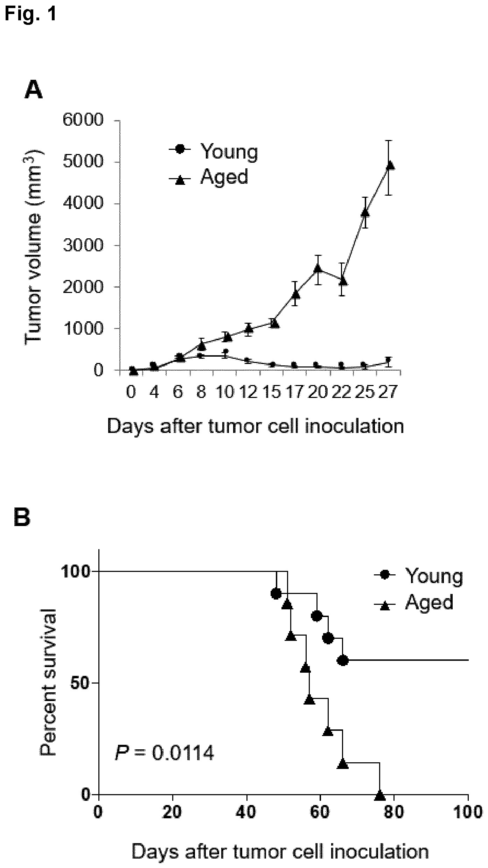

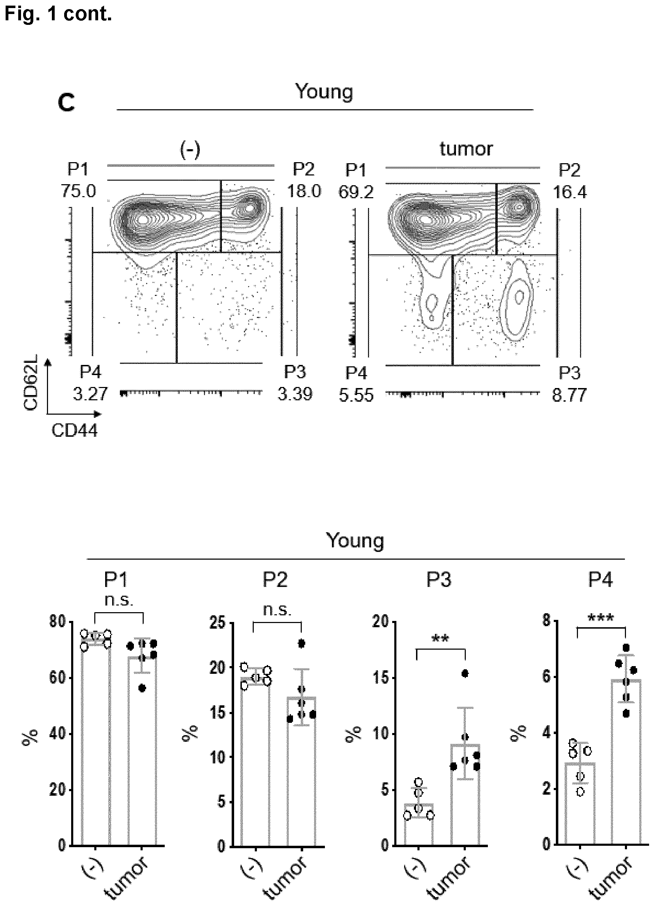

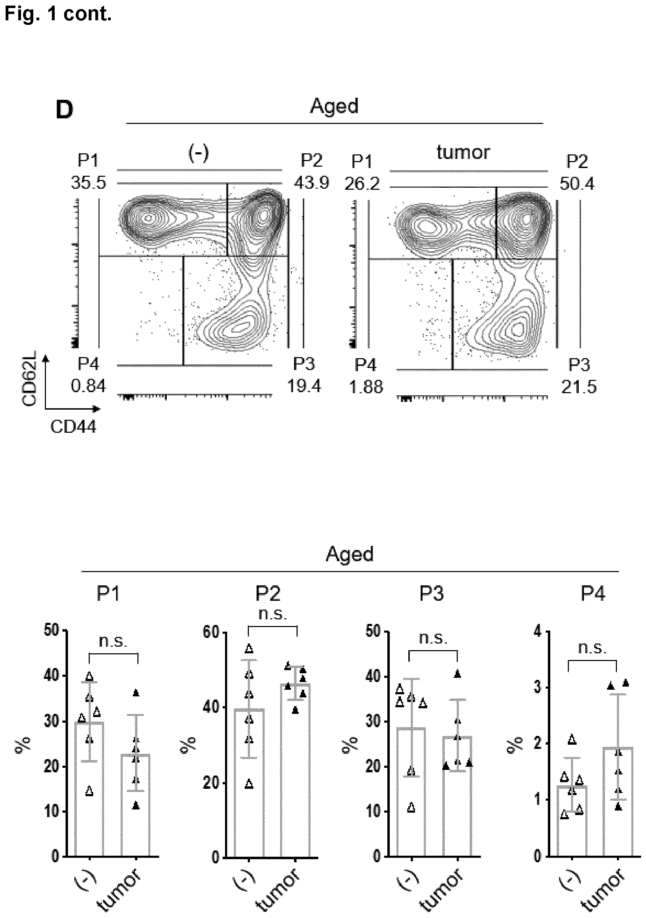

[Fig. 1] Loss of anti-tumor activity and reduced P4 cell induction in aged PD-1 KO mice. (A and B) MC38 cells were i.d. (intradermally) inoculated into young and aged PD-1 KO mice. (A) MC38-tumor sizes in young (3-4 month old) and aged (15 month old) PD-1 KO mice. (B) Kaplan-Meier plot of percent survival of MC38-tumor-bearing PD-1 KO mice. (C and D) Analysis of CD8⁺ T-cell subsets in young (C, 2-3 month old) or aged (D, 15-21 month old) PD-1 KO mice with or without inoculation of MC38 cells. Stained peripheral lymph node (PLN) and draining lymph node (DLN) cells on day 9. Representative plots showing expression of CD44 and CD62L in CD3⁺CD8⁺ T cells and percentage of CD8⁺ T-cell subsets; CD44^{low}/CD62L^{high} (naive; P1), CD44^{high}/CD62L^{high} (central memory; P2), CD44^{high}/CD62L^{low} (effector/memory; P3), CD44^{low}/CD62L^{low} (P4). P values were calculated by log-rank test or Student's t-test. *p < 0.05; **p < 0.01; n.s., not significant. Data are presented as the mean ± SEM of mice (n = 8-10 for A and B; n = 5-6 for C and D).

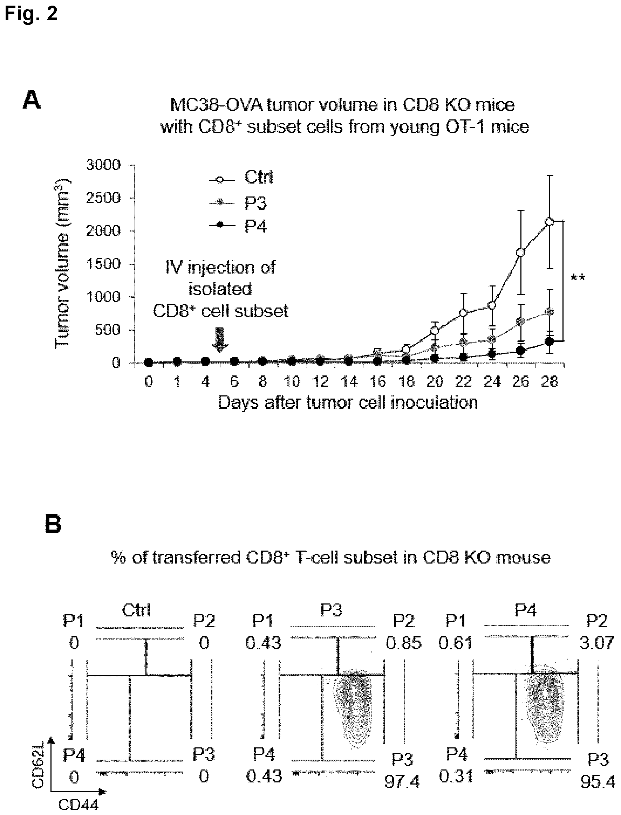

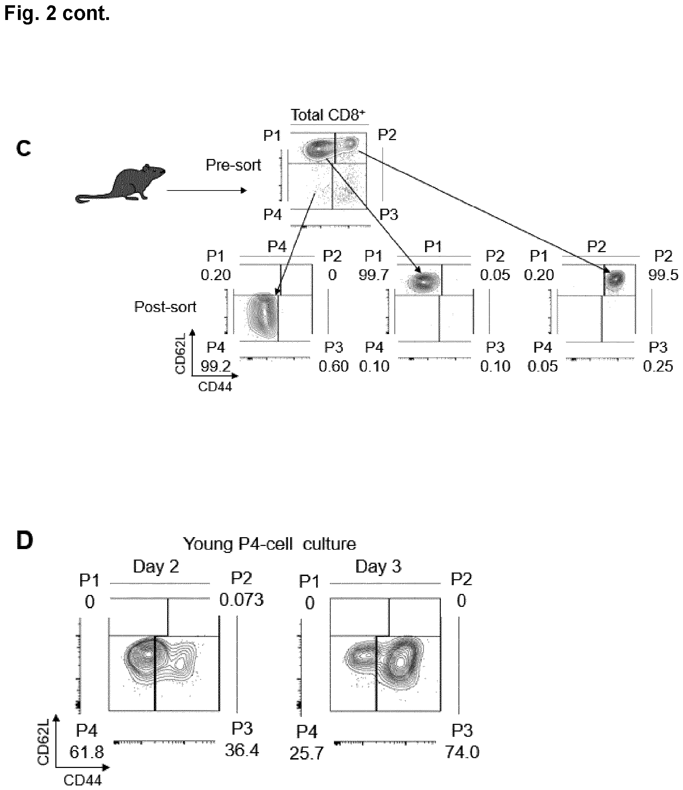

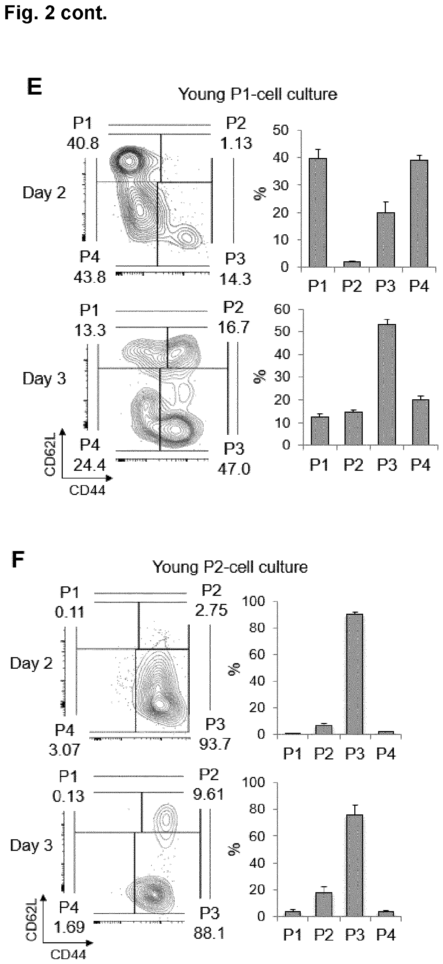

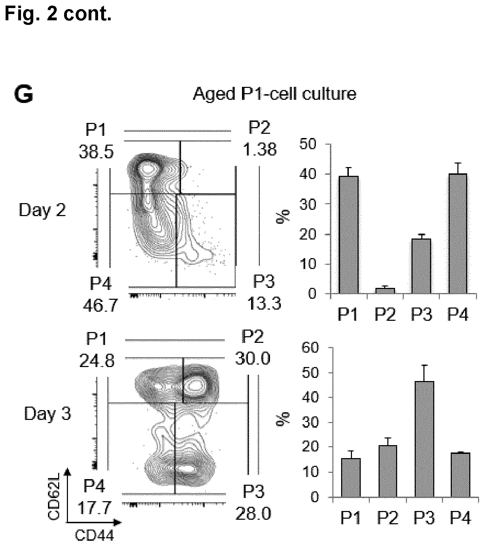

[Fig. 2] Anti-tumor activity of P4 cells through differentiation to P3 cells. (A and B) To obtain the OT-I P3 and P4 subsets, MC38-OVA cells were injected into the tail vein of young OT-1 mice, and each subset was isolated from splenocytes. The P3 or P4 subset cells were adoptively transferred into CD8 KO mice 5 days after intradermal injection of MC38-OVA cells. (A) Tumor volume in MC38-OVA-bearing CD8 KO mice with or without the transfer of P3 or P4 cells. (B) FACS analysis of the transferred CD8⁺ T-cell subsets in peripheral blood on day 11. (C) Scheme of CD8⁺ T-cell subset isolation from splenocytes of young PD-1 KO mice. (D to G) FACS analysis of P4 (D), P1 (E) or P2 (F) cells isolated from young (2-3 month old) or aged (16-18 month old) PD-1 KO mice, stimulated with anti-CD3/CD28 antibodies and IL-2, and then cultured for 2 or 3 days. Representative plots showing expression of CD44 and CD62L in the indicated culture cells and percentages of CD8⁺ T-cell subsets. Data are presented as the mean ± SEM of mice (n = 3-5); **p < 0.01 (ANOVA followed by Tukey's test).

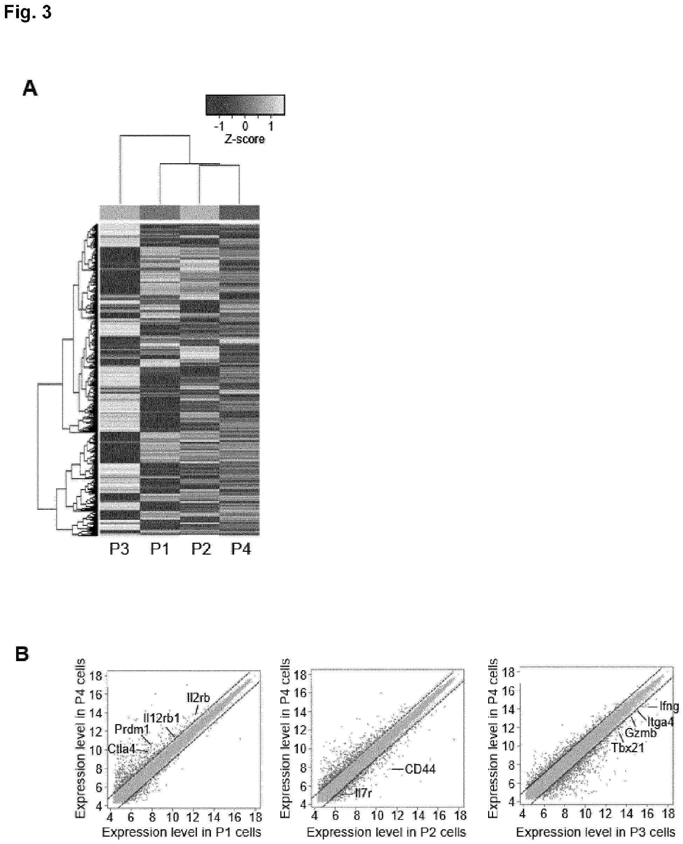

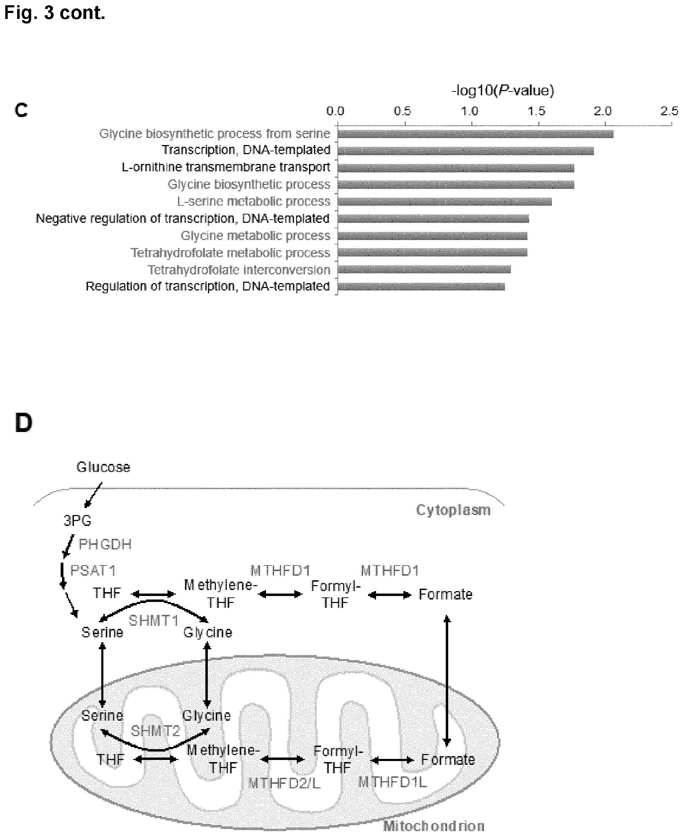

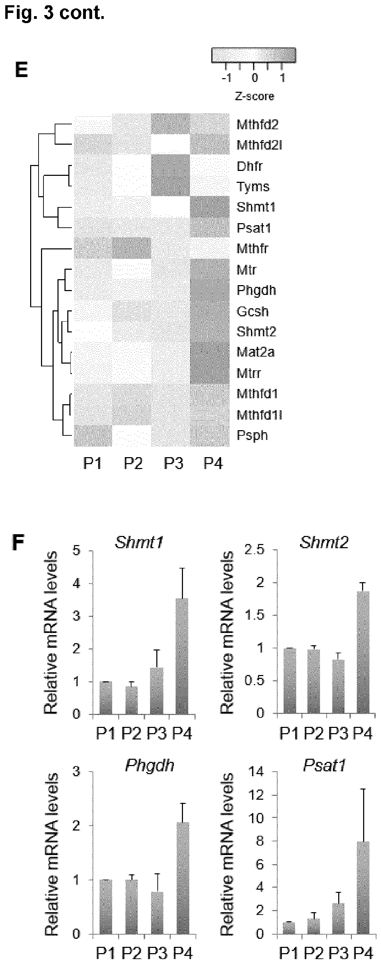

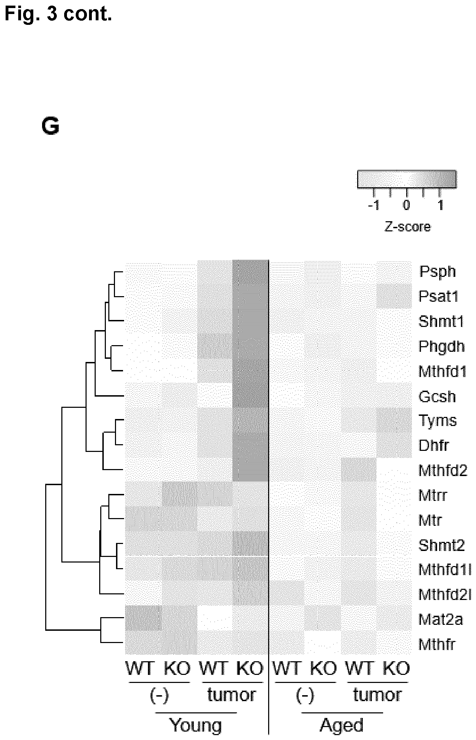

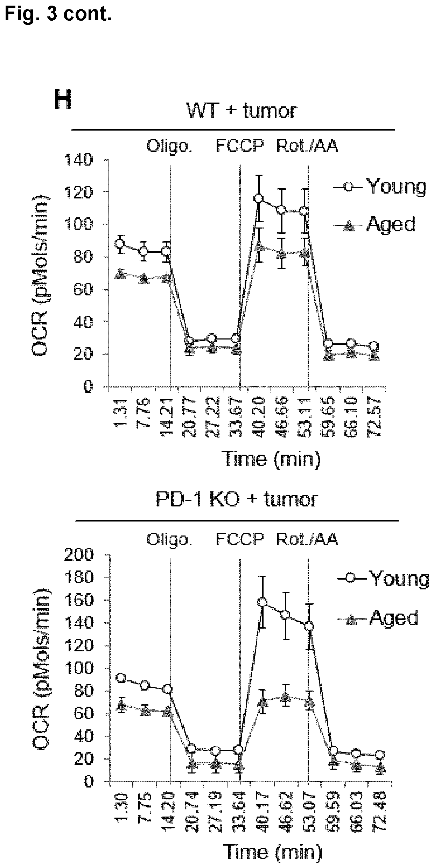

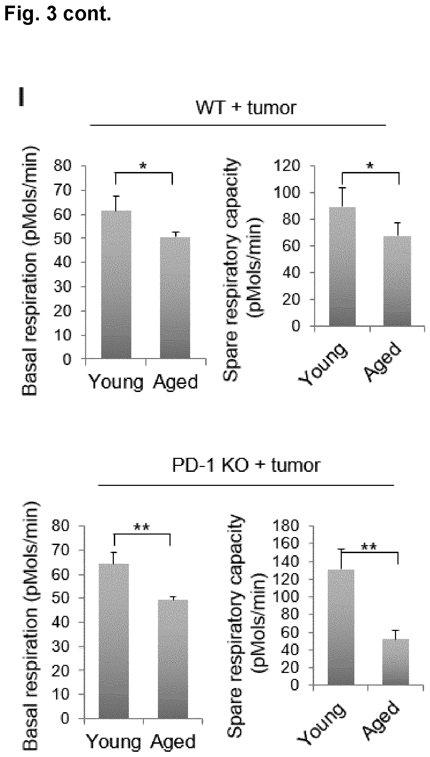

[Fig. 3] Increased expression of 1C-metabolism-related genes in P4 cells. (A to E) Microarray analysis of P1, P2, P3, and P4 cells isolated from young PD-1 KO mice (1-3 month old; 9 mice pooled). (A) Hierarchical clustering heat map of all detected genes. (B) Scatter plots represent normalized log intensities of individual probes. Dashed lines indicate log 2-fold change. Genes previously linked to CD8⁺ T-cell activation and differentiation are listed. (C) Top 10 enriched gene ontology (GO) terms from the upregulated genes in P4 cells. GO terms involved in 1C metabolism are highlighted in red. (D) Schematic of 1C metabolic pathways. THF: tetrahydrofolate. (E) Heat map showing the expression of 1C-metabolism-related genes in CD8⁺ T-cell subsets. (F) Relative mRNA expression of 1C-metabolism-related genes in CD8⁺ T-cell subsets from splenocytes of young PD-1 KO mice. (G) Microarray analysis of WT or PD-1 KO CD8⁺T cells in PLNs or DLNs of young (2 month old, 3 mice pooled) or aged (17 month old, 6 mice pooled) mice with or without MC38 tumor (day 9). Heat map shows the expression of 1C-metabolism-related genes in the indicated cells. (H and I) Oxygen consumption rates (OCR) of WT or PD-1 KO CD8⁺ T cells from DLNs of young (2-3 month old) or aged (15-19 month old) mice were measured using a Seahorse XFe96 analyzer. OCR trace (H), and basal respiration and spare respiratory capacity were calculated from OCR values (I). *p < 0.05; ** p < 0.01 (Student's t-test). Data are presented as the mean ± SEM (n = 4).

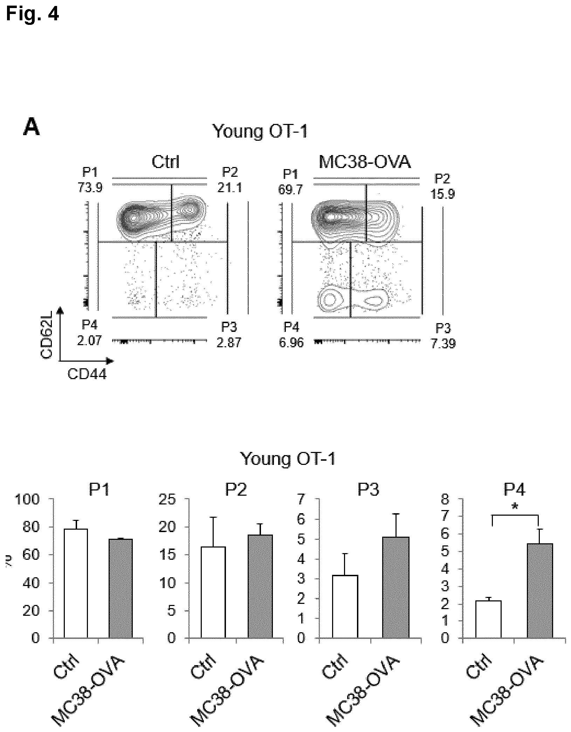

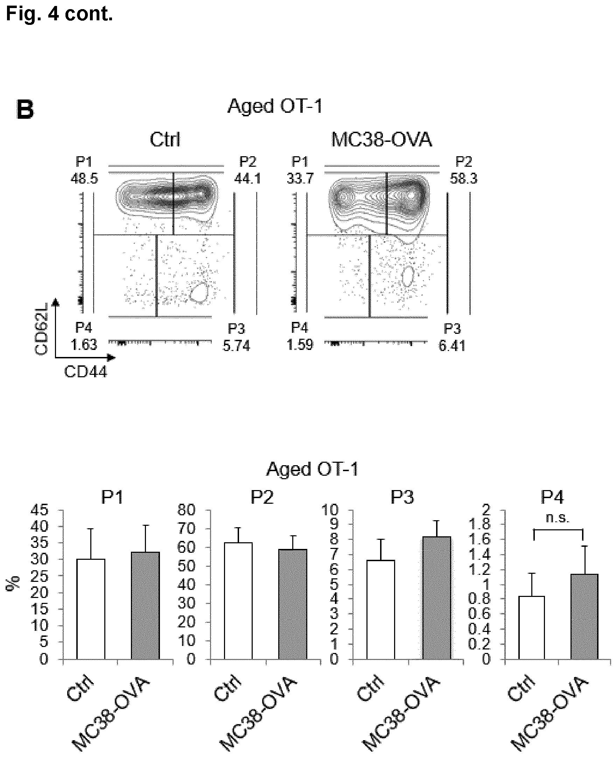

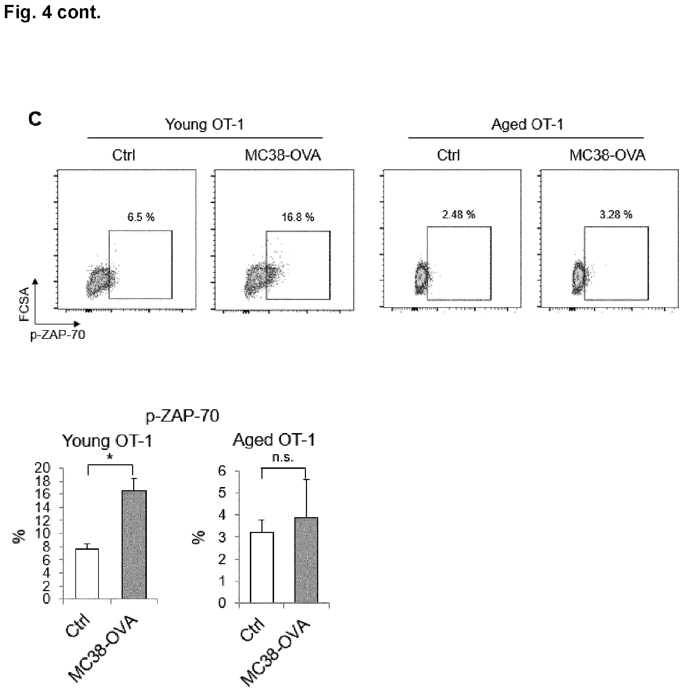

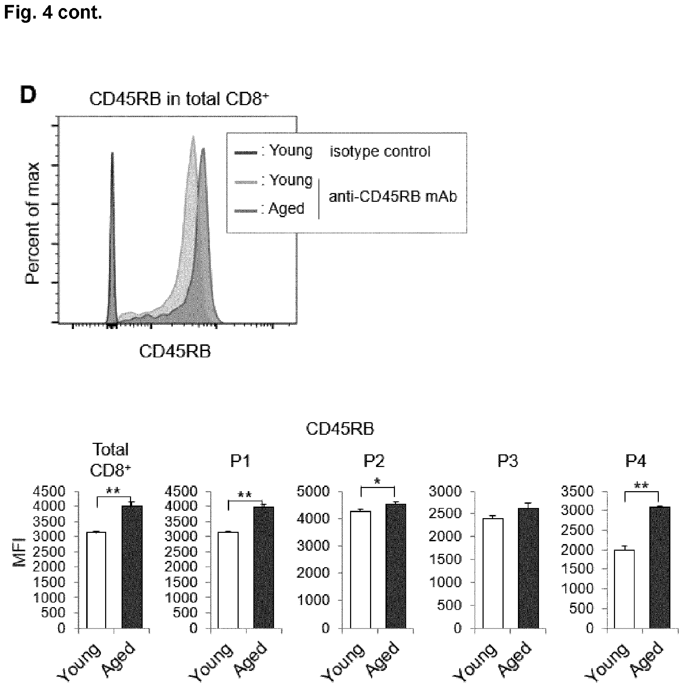

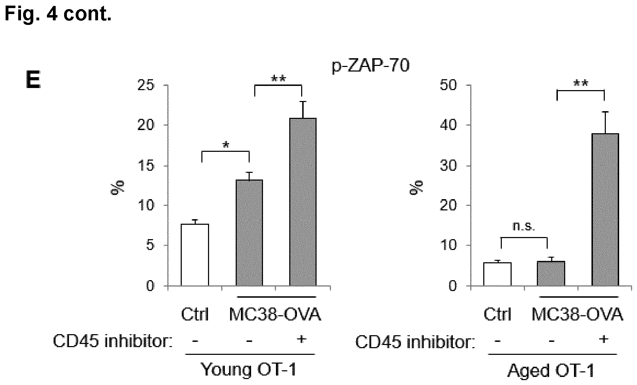

[Fig. 4] P4 cell induction in aged CD8⁺ T cells is attenuated by TCR signaling inhibition via high CD45RB expression. (A and B) Analysis of CD8⁺ T-cell subsets in DLNs of young (2-3 month old) (A) or aged (12-15 month old) (B) OT-1 mice with or without injection of MC38-OVA cells into the tail vein. Representative plots showing CD44 and CD62L expression on CD8⁺ T cells and the percentage of each CD8⁺ T-cell subset in the indicated mice 5 days after MC38-OVA injection. (C) The percentages of pZAP70⁺ CD8⁺ T cells in PLNs from young or aged OT-1 mice with or without MC38-OVA injection. (D) Representative plots of CD45RB expression levels in total CD8⁺ T cells or each subset from young (2-3 month old) or aged (14-17 month old) OT-1 mice, and fluorescence intensities (MFI). (E) CD8⁺ T cells were isolated from PLNs of young or aged OT-1 mice and cultured with or without MMC-treated MC38-OVA cells for 1 day. In the last 1 hour of culture, vehicle or CD45 inhibitor (0.05 µM) was added. Data are represented as the mean ± SEM (n = 3-6). *p < 0.05; **p < 0.01; n.s., not significant (ANOVA followed by Tukey's test or Student's t-test).

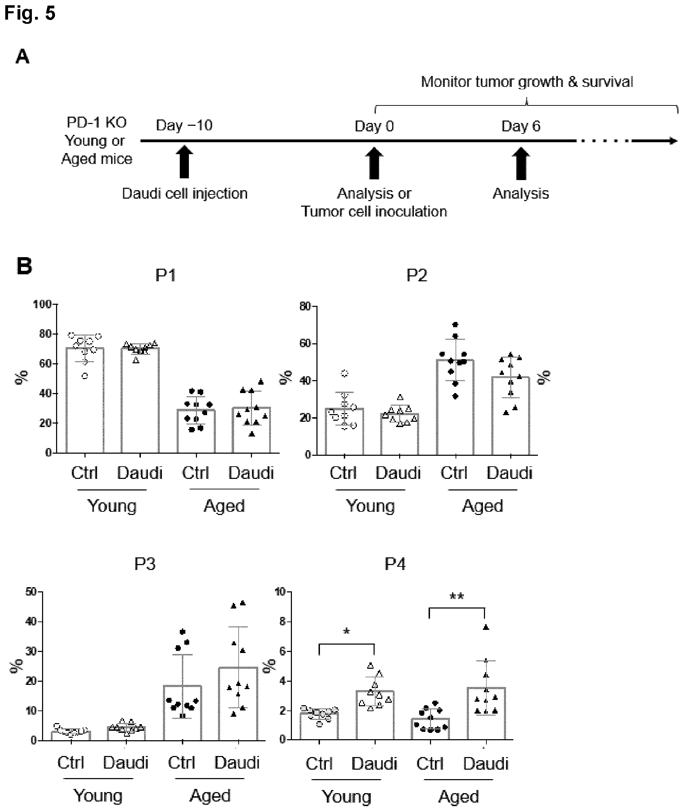

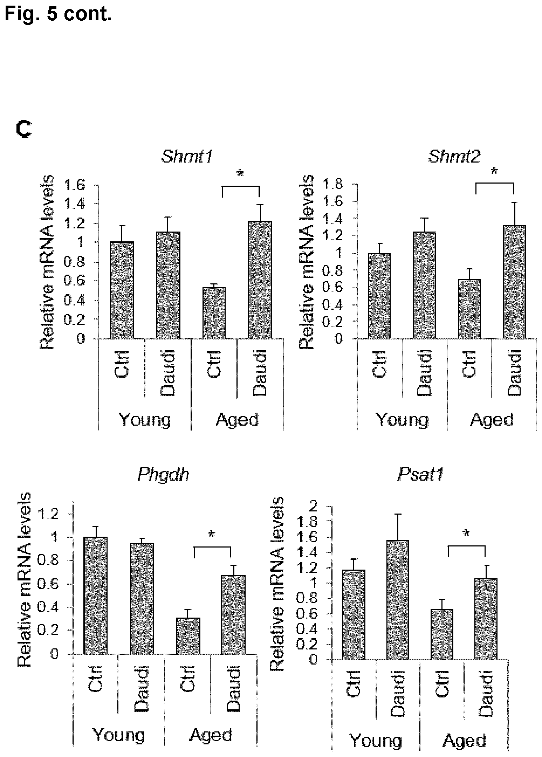

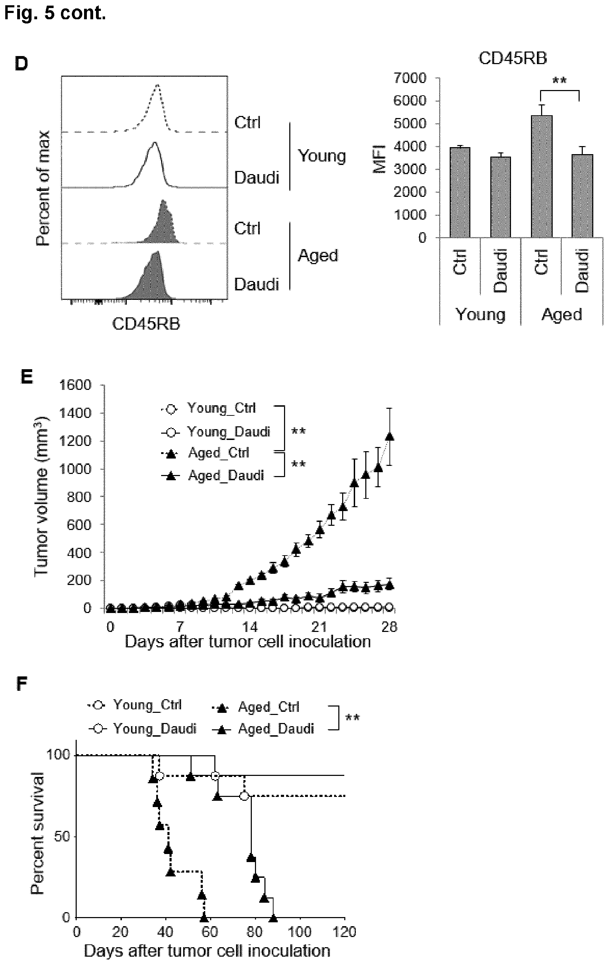

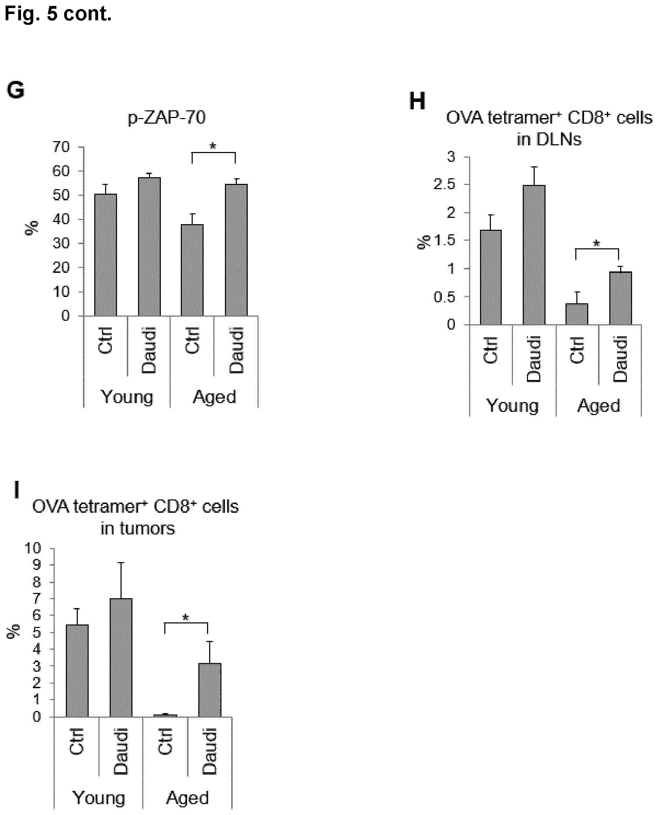

[Fig. 5] Recovery of P4 cell induction and anti-tumor activity in aged mice by xenogeneic cell injection. (A) Schematic diagram of the experimental schedule. (B to D) Young (3-4 month old) and aged (16-18 month old) PD-1 KO mice were injected with Daudi cells into the tail vein. Ten days after injection (day 0), PLN cells of the mice were analyzed by FACS and real-time PCR. (B) Percentages of P1-P4 subsets relative to all CD8⁺ T cells in PLNs. (C) Expression levels of 1C-metabolism-related genes in CD8⁺ T cells. (D) CD45RB expression levels in P1 cells from PLNs. (E to I) Young (3-4 month old) and aged (17-18 month old) PD-1 KO mice were injected with Daudi cells into the tail vein (day -10). Ten days after the injection (day 0), the mice were i.d. (intradermally) inoculated with MC38 (E-G) or MC38-OVA (H and I) cells and used in the following experiments. MC38-tumor sizes (E) and percent survival (F). (G) The percentage of pZAP70⁺ cells relative to all CD8⁺ T cells from DLNs on day 6. (H and I) Frequency of OVA tetramer⁺ CD8⁺ T cells in DLNs or tumor tissues on day 6. Data are presented as the mean ± SEM (n = 9-10 for B; n = 4-6 for C-I). *p < 0.05; **p < 0.01 (ANOVA followed by Tukey's test, log-rank test, or Student's t-test).

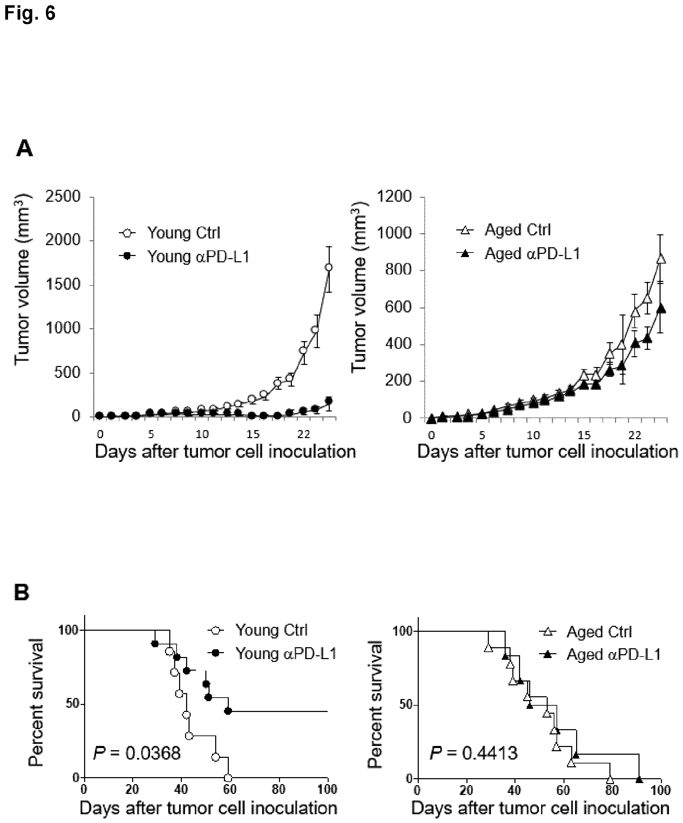

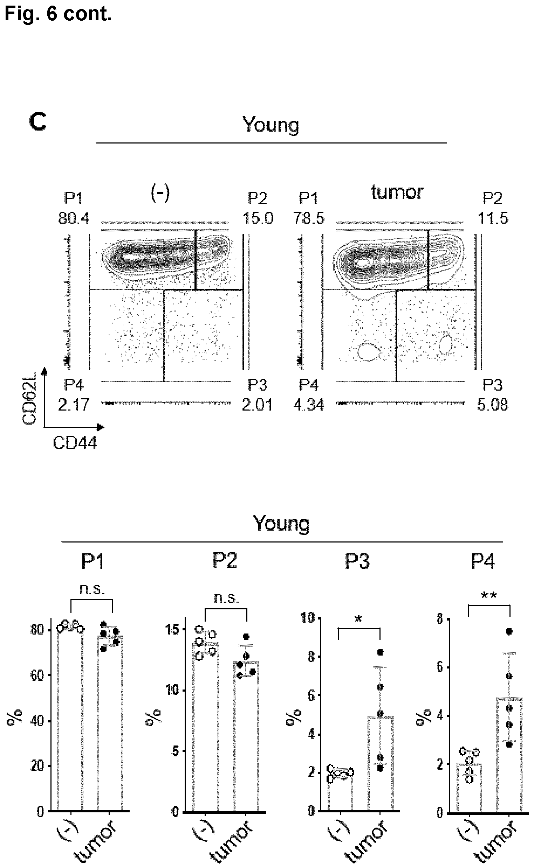

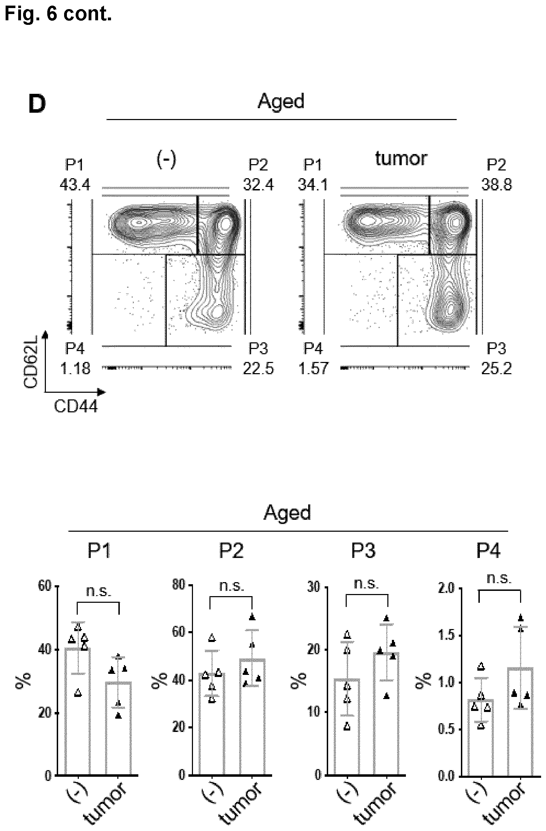

[Fig. 6] Loss of anti-tumor activity and reduced P4 cell induction in aged WT mice. (A and B) MC38 cells were i.d. inoculated into young (2 month old) and aged (14 month old) C57BL/6 WT mice. Then, those mice received administration of anti-PD-L1 antibody on days 5, 11, and 17. (A) MC38-tumor sizes in young and aged WT mice. (B) Kaplan-Meier plots of percent survival of MC38-tumor bearing WT mice. P values were calculated by log-rank test. (C and D) Analysis of CD8⁺ T-cell subsets in young (C, 2 month old) or aged (D, 15 month old) WT mice with or without injection of MC38 cells. Stained PLN and DLN cells on day 9. Representative plots showing expression of CD44 and CD62L in CD3⁺CD8⁺ T cells, and percentages of CD8⁺ T-cell subsets; CD44^{low}/CD62L^{high} (naive; P1), CD44^{high}/CD62L^{high} (central memory; P2), CD44^{high}/CD62L^{low} (effector/memory; P3), and CD44^{low}/CD62L^{low} (P4). *p < 0.05; **p < 0.01; n.s., not significant (Student's t test). Data are presented as the mean ± SEM of mice (n = 5 for A, C, and D; n = 9-11 for B).

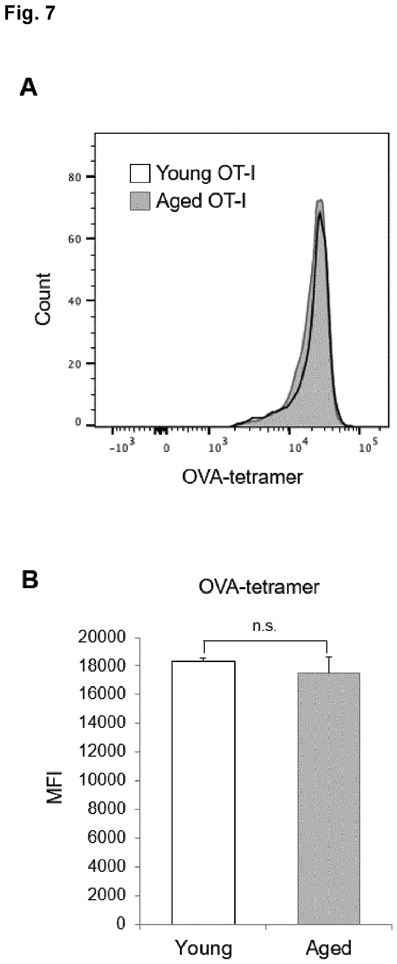

[Fig. 7] Similar TCR avidity to OVA antigen between young and aged OT-1 CD8⁺ T cells. Mean fluorescence intensity (MFI) of OVA-specific MHC tetramer in young and aged OT-1 mice is shown in representative plot (A) and graph (B). Data are presented as the mean ± SEM of mice (n = 4-6). n.s.: not significant (Student's t test).

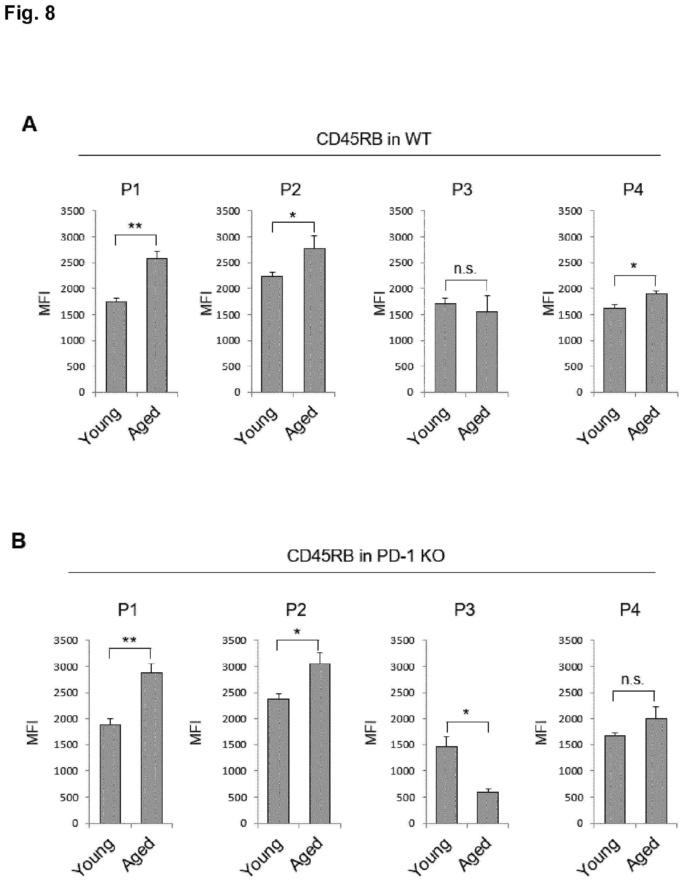

[Fig. 8] Enhanced CD45RB expression in CD8⁺ T cells from aged WT or PD-1 KO mice. CD45RB expression levels in CD8⁺ T-cell subsets from PLNs of WT (A) or PD-1 KO (B) mice were measured by FACS analysis using anti-CD45RB antibody. Data are presented as the mean ± SEM of mice (n = 3 - 6). *p < 0.05; **p < 0.01; n.s., not significant (Student's t test).

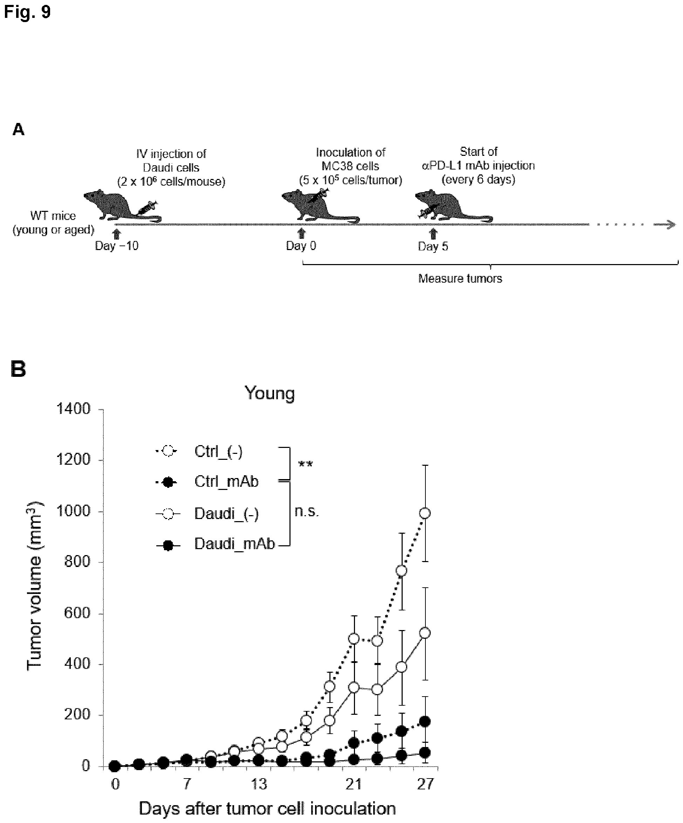

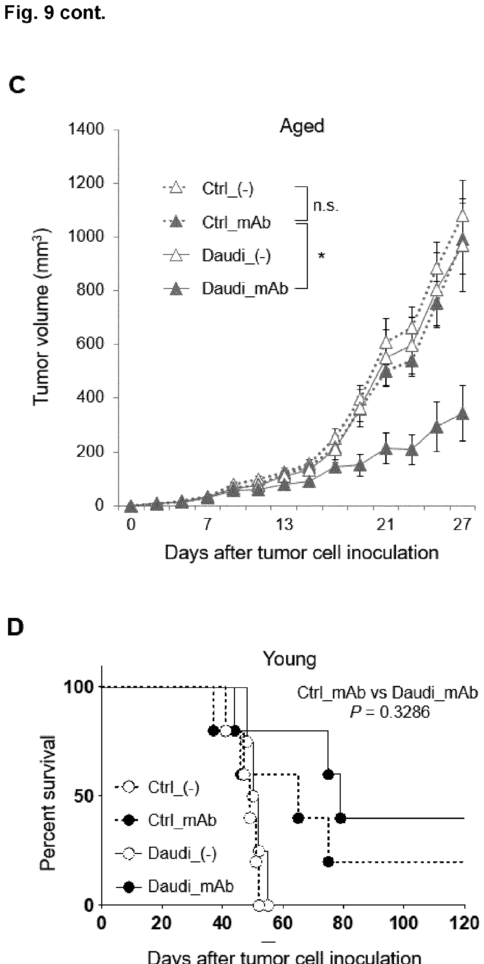

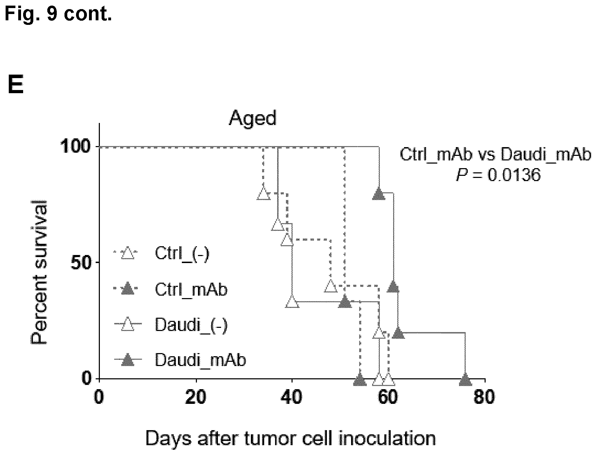

[Fig. 9] Amelioration of age-related unresponsiveness to PD-1 blockade therapy in aged WT mice by xenogeneic cell injection. Daudi cells were i.v. injected into young (1 month old) and aged (17-19 month old) C57BL/6 WT mice. Mice without Daudi cell injection were used as control (Ctrl). Ten days after the injection, the mice were i.d. inoculated with MC38 cells. Administration of anti-PD-L1 antibody was started on day 5, and repeated 3 times at an interval of 6 days. (A) Schematic diagram of the experimental schedule. (B and C) MC38-tumor sizes in young (B) and aged (C) WT mice. (D and E) Percent survival of MC38-tumor bearing young (D) and aged (E) WT mice. Data are presented as the mean ± SEM of mice (n = 5-6). *p < 0.05; **p < 0.01; n.s., not significant (ANOVAfollowed by Tukey's test or log-rank test).

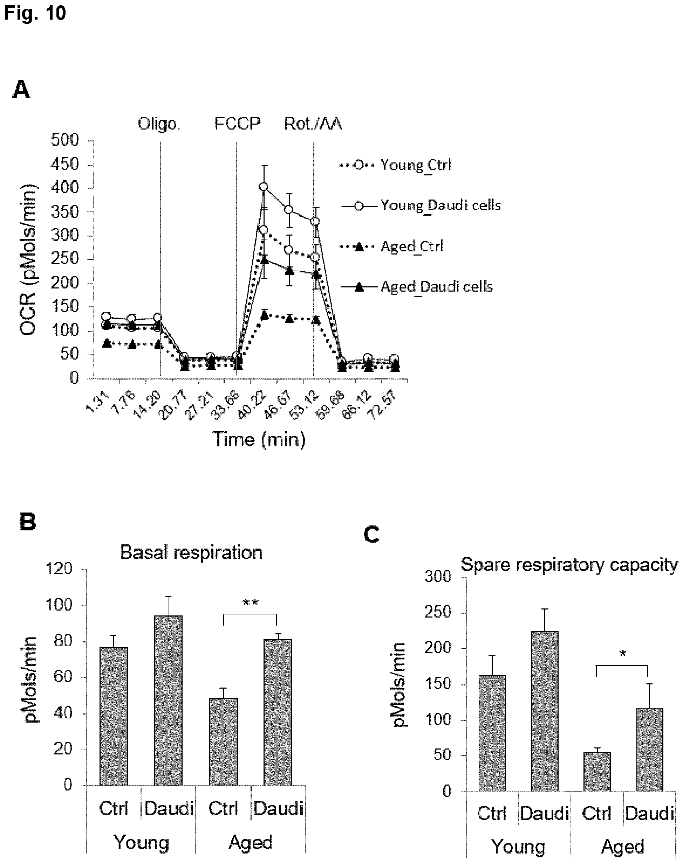

[Fig. 10] Recovery of age-induced mitochondrial disorder by xenogeneic cell injection. MC38-OVA cells were i.d. inoculated into young (3-4 month old) and aged (17-18 month old) PD-1 KO mice which received injection of PBS (Ctrl) or Daudi cells 10 days ago. Six days after MC38-OVA inoculation, OCR of CD8⁺ T cells isolated from DLNs of the indicated mice was measured using a Seahorse XFe96 analyzer. OCR trace (A), basal respiration (B) and spare respiratory capacity (C) were calculated from OCR values. Data are presented as the mean ± SEM (n = 3). *p < 0.05; **p < 0.01 (ANOVA followed by Tukey's test).

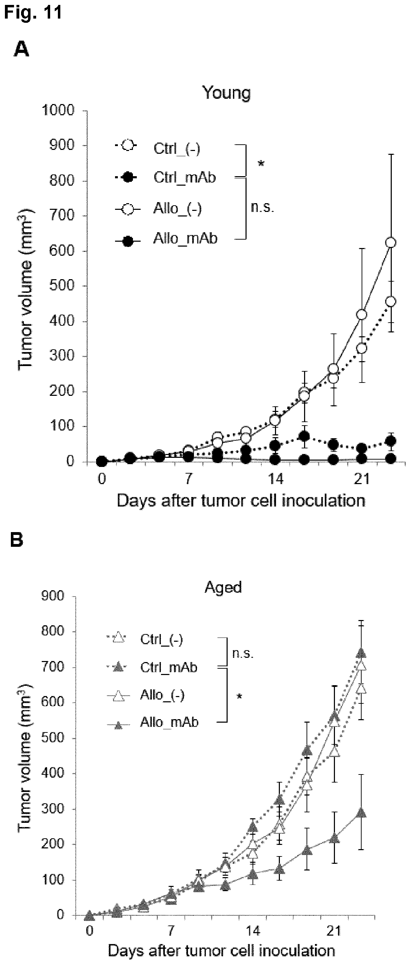

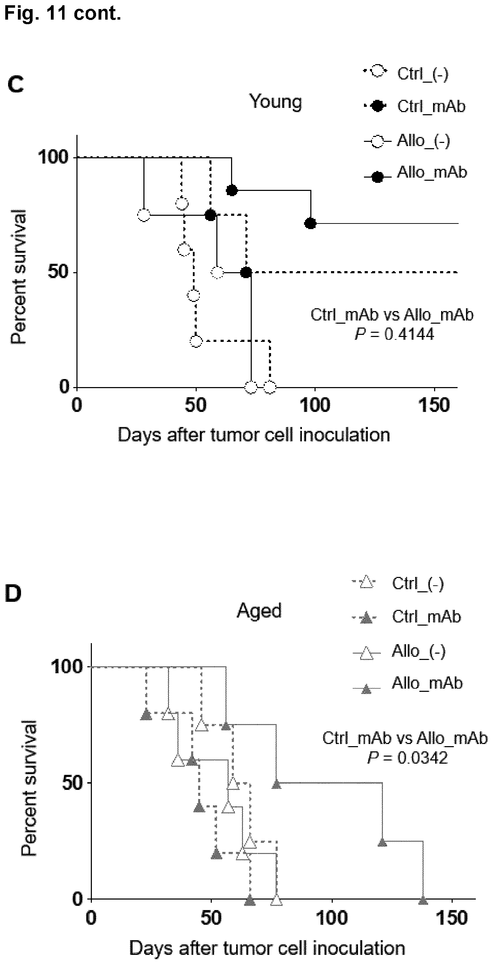

[Fig. 11] Rescue of age-induced resistance to PD-1 blockade therapy by allogeneic cell injection. Mitomycin C-treated splenocytes from C57BL/6 (Ctrl) or Balb/c (Allo) mice were injected into the tail vein of young (1 month old) and aged (14-18 month old) C57BL/6 WT mice. Subsequent experimental conditions were the same as shown in Fig. 9A. MC38-tumor sizes in young (A) and aged (B) C57BL/6 WT mice. Data are presented as the mean ± SEM (n = 3). *p < 0.05; n.s., not significant (ANOVA followed by Tukey's test). (C and D) Percent survival of MC38-tumor-bearing young (C) and aged (D) C57BL/6 WT mice. P values were calculated by log-rank test (n = 5-6).

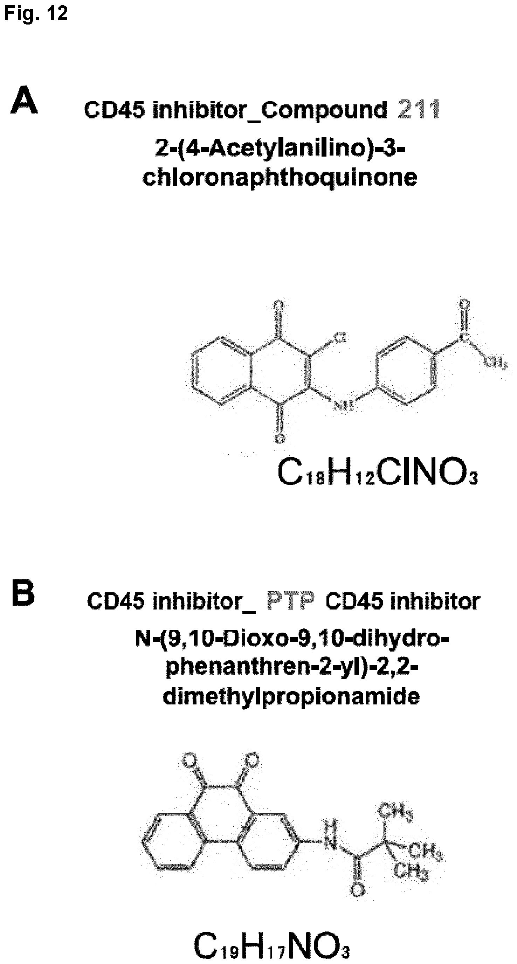

[Fig. 12] Designations and structural formulas of CD45 inhibitors. A) 2-(4-acetylanilino)-3-chloronaphthoquinone; hereinafter, sometimes referred to as "211". B) N-(9,10-dioxo-9,10-dihydrophenanthren-2-yl)-2,2-dimethylpropionamide; hereinafter, sometimes referred to as "PTP".

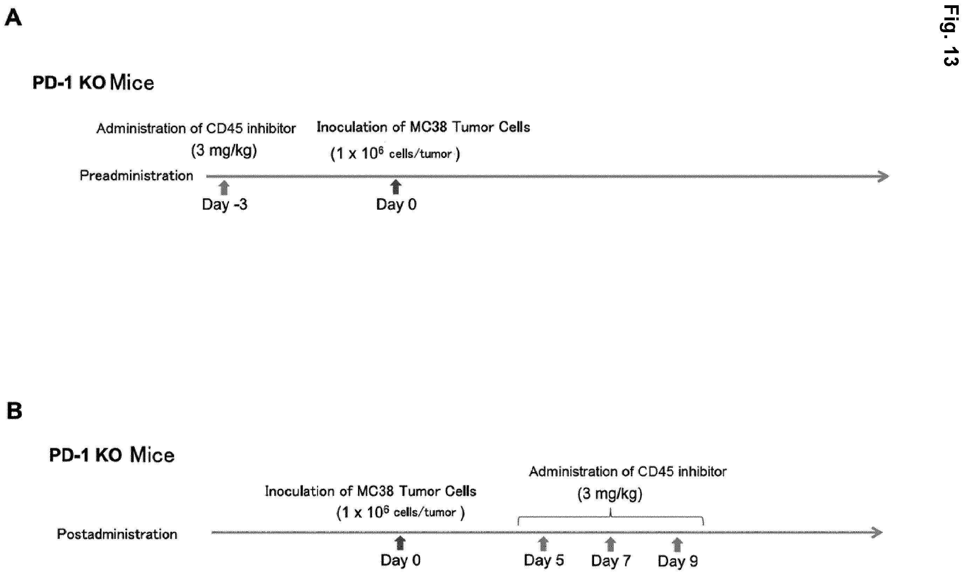

[Fig. 13] Experimental method and flow chart.

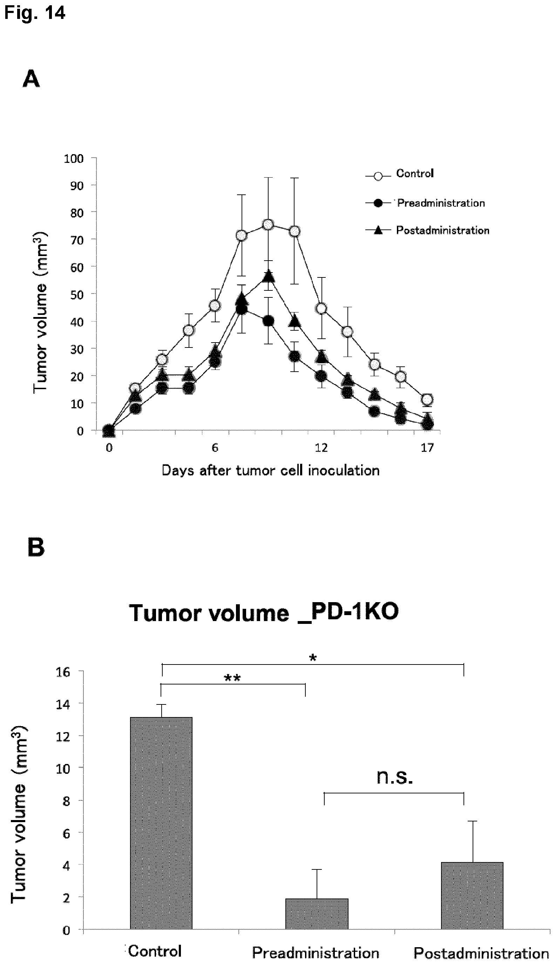

[Fig. 14] Improvement of PD-1 blockade effect by CD45 inhibition. Preadministration: Vehicle (control) or 211 was administered to PD-1 KO mice, into which MC38 cells (1 × 10⁶) were inoculated after 3 days. Postadministration: From 5 days after MC38 cell inoculation to PD-1 KO mice (i.e. from Day 5), 211 was administered three times in total at an interval of two days. A) Tumor volume growth curve; B) Tumor volume 17 days after MC38 cell inoculation. Data are presented as the mean ± SEM of 5 mice. *p < 0.05; **p < 0.01; n.s., not significant

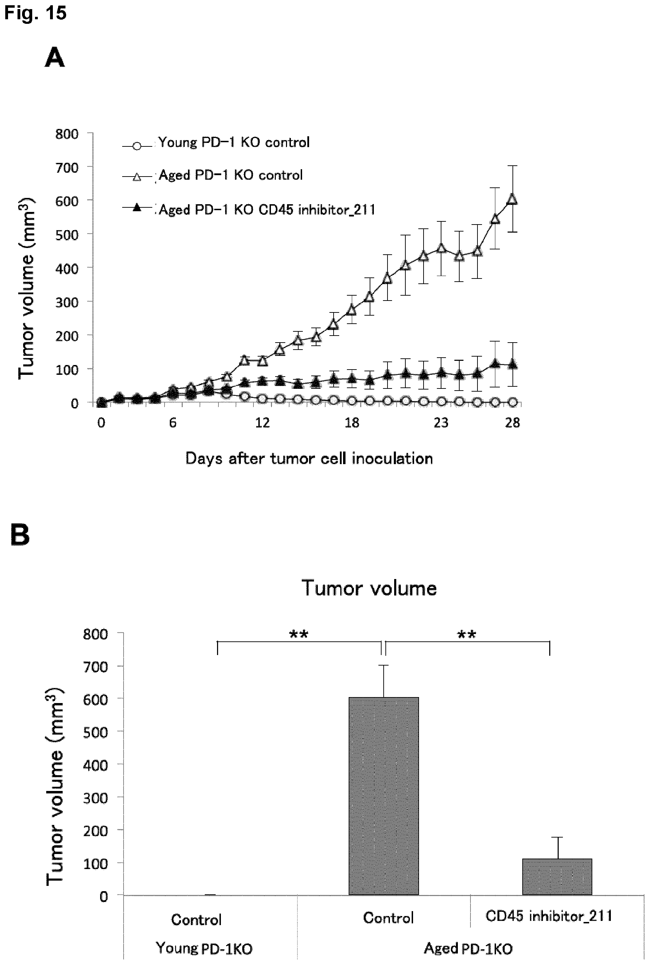

[Fig. 15] Increased effect on age-related PD-1 blockade resistance by CD45 inhibition. A CD45 inhibitor 211 was administered to juvenile (1-2 month old) or aged (14-16 month old) PD-1 KO, into which MC38 cells were inoculated after 3 days. A) Tumor volume growth curve; B) Tumor volume 28 days after MC38 cell inoculation. Data are presented as the mean ± SEM of 6 mice. **p < 0.01.

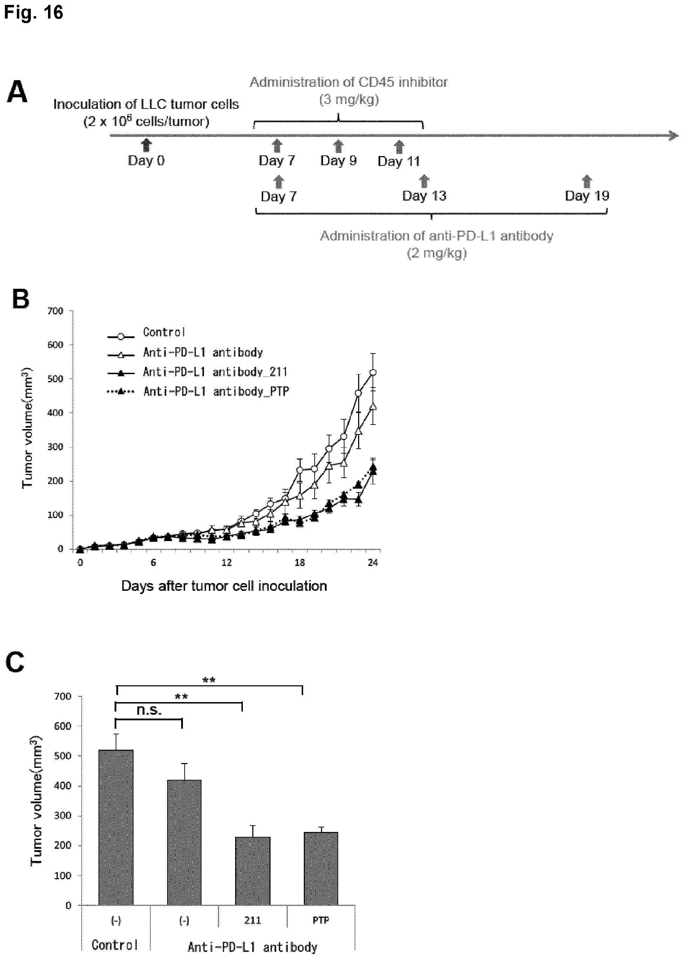

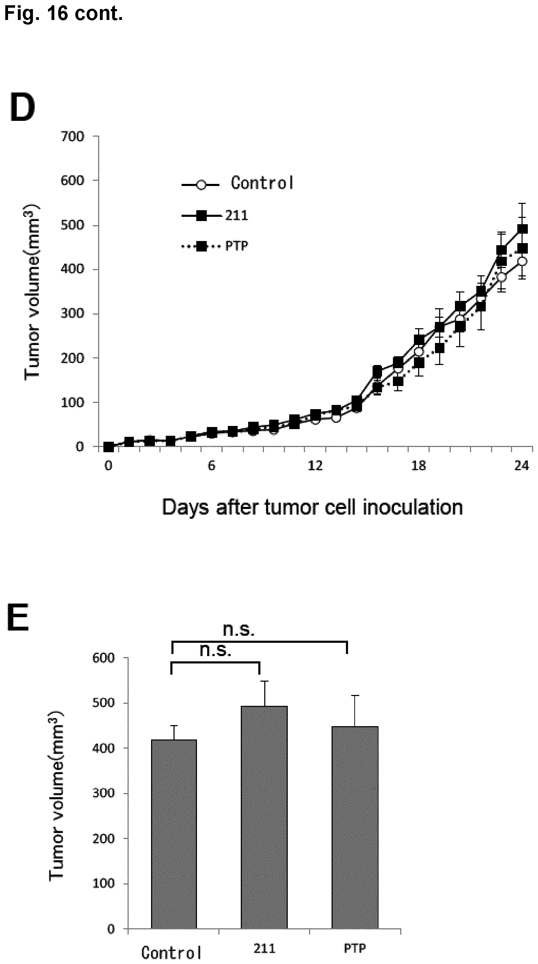

[Fig. 16] Increased effect on PD-1 blockade-resistant cancer by CD45 inhibition. A) Experimental method and flow chart. A CD45 inhibitor 211or PTP was administered to juvenile (1-2 month old) WT mice from 7 days after inoculation of LLC cells showing resistance to immunotherapy (i.e. from Day 7) three times in total at an interval of 2 days. Anti-PD-L1 antibody was administered three times in total from Day 7 at an interval of 6 days. B and D) Tumor volume growth curve, C and E) Tumor volume 24 days after MC38 cell inoculation. Data are presented as the mean ± SEM of 5 to 7 mice. **p < 0.01; n.s., not significant.
[Fig. 17] Model diagram of TCR signal transduction.

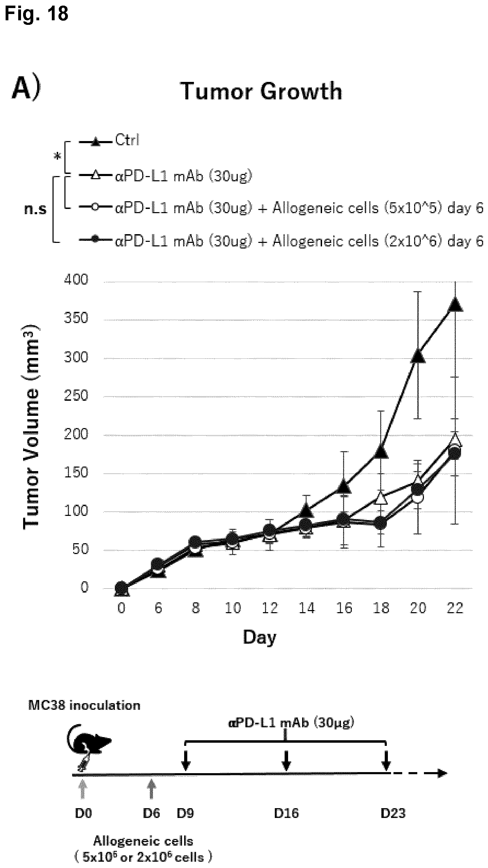

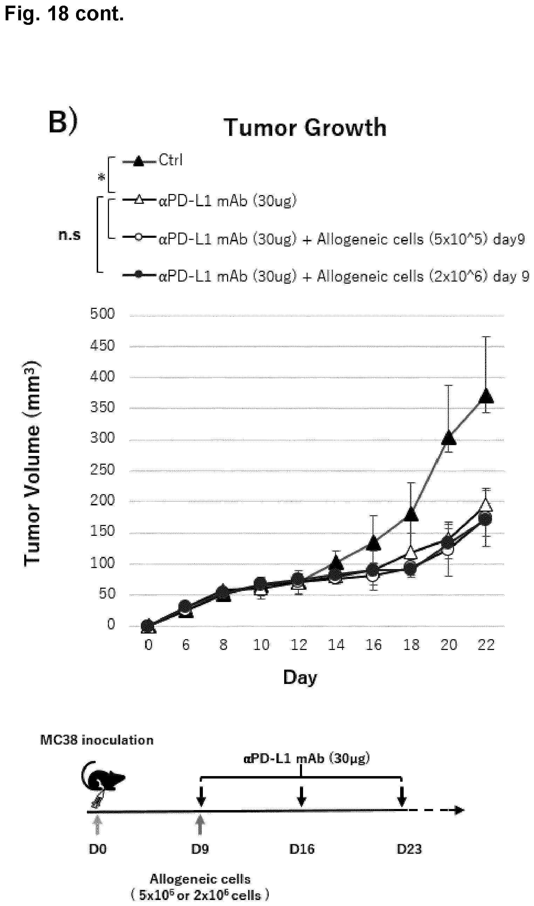

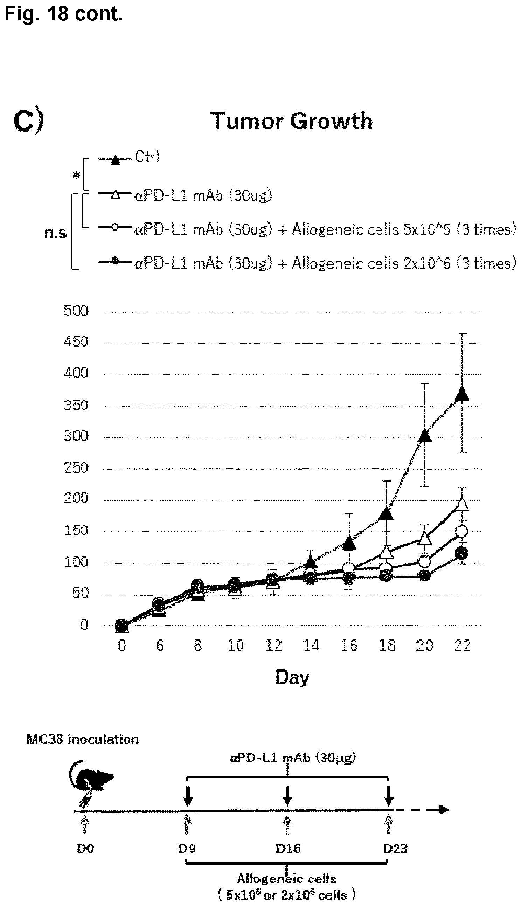

[Fig. 18] Efficacy of immune checkpoint inhibitor is enhanced by administration of allogeneic cells. MC38 cancer cells (5 × 10⁵ cells) were i.d. injected into young (6 month old) C57BL/6 mice. Nine, 16 and 23 days after the injection, anti-PD-L1 antibody (clone 1-111A.4) (1.5 mg/kg) was administered intraperitoneally. Further, mitomycin C-treated splenocytes from Balb/c mice (allogeneic) (5 ×10⁵ cells/mouse or 2 × 10⁶ cells/mouse) were intravenously injected (A) only once after 6 days, (B) only once after 9 days, or (C) three times in total after 9, 16 and 23 days, and chronological changes in tumor size were measured. Points in each line graph are mean values from 5 to 6 mice, and error bars represent standard error. * means p-value less than 0.05, and n.s. means not significant (comparison was made by Turkey's test after one-way ANOVA).

### Description of Embodiments for Carrying out the Invention

Hereinbelow, the present invention will be described more specifically.

The present invention provides a pharmaceutical composition which comprises a substance capable of enhancing T cell receptor (TCR) signaling and is administered before, after or simultaneously with the administration of a PD-1 signaling inhibitor.

The T cell receptor (TCR) is an antigen receptor molecule expressed on the surface of the cell membrane of T cells. When an antigen binds to TCR, signals are transduced into T cells through phosphorylation of downstream factors such as Lck and ZAP-70, which causes activation of T cells.

Enhancement of TCR signaling may be confirmed by evaluating the phosphorylation of ZAP-70 in CD8⁺ T cells under antigen stimulation. When the phosphorylation level of ZAP-70 is high, it can be said that TCR signaling is enhanced. The phosphorylation of ZAP-70 may be detected with anti-p-ZAP-70 antibody by flow cytometry analysis.

It is possible to enhance the antitumor immunity effect of CD⁸ T cells by enhancing TCR signaling. Therefore, by enhancing TCR signaling, it is possible to enhance the antitumor effect of PD-1 blockade therapy, which could result in producing therapeutic effect on aged animals or human beings showing resistance to PD-1 blockade and even on resistant cancers.

As examples of substances capable of enhancing TCR signaling, CD45 inhibitors and/or cells may be enumerated. These substances may also produce an effect of inducing P4 cells. P4 cells represent a CD8⁺T cell subset which is CD44^{low}CD62L^{low}. In mouse, three major CD8⁺ T cell subsets are defined using surface markers CD44, CD62L (L-selectin), etc.; naive (also called P1; CD44^{low}CD62L^{high}), central memory (P2; CD44^{high}CD62L^{high}), and effector/memory (P3; CD44^{high}CD62L^{low}). The remaining CD44^{low}CD62L^{low} (P4) CD8⁺ T cell subunit is a very minor population.

As examples of CD45 inhibitors, 2-(4-acetylanilino)-3-chloronaphthoquinone, N-(9,10-dioxo-9,10-dihydrophenanthren-2-yl)-2,2-dimethylpropionamide, and analogs thereof may be enumerated.

As used herein, "analog" is a concept encompassing salts of compounds, derivatives of compounds, prodrugs to active metabolites, active metabolites to prodrugs, solvates thereof, and so on.

As salts of compounds, those salts which are formed with acids such as hydrochloric acid, mesylate (methanesulfonic acid), fumaric acid, phosphoric acid, etc. may be enumerated when compounds have amino groups (including substituted amino groups) or amide groups.

Derivatives of compounds are compounds which have undergone minor modifications that would not greatly change the structure or property of the original compounds. Such minor modifications include, but are not limited to, introduction or substitution of functional groups, oxidation, reduction, and substitution of atoms. As derivatives of 2-(4-acetylanilino)-3-chloronaphthoquinone, the compound in which chloro (-Cl) group is substituted with other halogen group (e.g., fluoro (-F) group, bromo (-Br) group, etc.) and the compound in which acetyl (CH₃CO-) group is substituted with other functional group (e.g., hydroxy group, amino (-NH₂) group, pivaloyl group, benzoyl group, etc.) may be given. As derivatives of N-(9,10-dioxo-9,10-dihydrophenanthren-2-yl)-2,2-dimethylpropionamide, the compound in which dimethylpropionyl group is substituted with other alkyl group (e.g., methyl group, ethyl group, propyl group, benzyl group, etc.) may be given.

Examples of prodrugs include, but are not limited to, compounds in which an amino group of the active compound is acylated, alkylated or phosphorylated (e.g., an amino acid of the active compound is eicosanoylated, alanylated, pentylaminocarbonylated, (5-methyl-2-oxo-1,3-dioxolen-4-yl)methoxycarbonylated, tetrahydrofuranylated, pyrrolidyl methylated, pivaloyloxymethylated, tert-butylated, or the like); and compounds in which an amide group of the active compound is alkylated (e.g., methylated, ethylated, propylated, or the like).

Examples of solvates include, but are not limited to, solvates with solvents such as water, methanol, ethanol, acetonitrile, and so on. The solvents may be single ones or mixtures of two or more solvents.

A cell capable of enhancing TCR signaling may be either a xenogeneic or allogeneic cell with respect to the target human or animal subj ect to which the pharmaceutical composition is to be administered. Alternatively, the cell may be a combination of xenogeneic and allogeneic cells. In Examples described later, xenogeneic Daudi cells and allogeneic splenocytes were injected into mice. As used herein, xenogeneic cell refers to a cell derived from another species; and allogeneic cell refers to a cell that is derived from the same species but different in HLA (Human leukocyte antigen) or MHC (major histocompatibility complex).

Various cells (xenogeneic or allogeneic) and tissues are known that are administered to living bodies for prevention or treatment of diseases. In the present invention, such cells may be used. For example, allogeneic cells such as breast cancer cell lines, chronic myelogenous leukemia cell lines, lung cancer cells, non-small cell lung cancer cell lines, melanoma cell lines, monocytes, pancreatic cancer cell lines, prostate cancer cell lines, renal cancer cell lines, and so on are used for treatment of various cancers (Human Vaccines & Immunotherapeutics 10:1, 52-63; January 2014). These cells may conveniently be radiation treated to prevent proliferation. Alternatively, these cells may be genetically modified so that they would secrete immune activation factors such as GM-CSF. Further, clinical trials are conducted in which melanoma patients are injected with xenogeneic cells such as mouse B 16 melanoma cells or Lewis lung cancer (LLC) cells (Eur J Dermatol 2016 Apr 1;26(2): 138-43). In addition to the above-listed cells, the following cells may also be used: cells used in blood preparations for transfusion (erythrocyte preparations, platelet preparations, whole blood preparations) and such preparations *per se,* as well as hematopoietic stem cells used in bone marrow transplantation. These cells may be either allogeneic or xenogeneic cells.

As used herein, the term "PD-1 signaling" refers to the signal transduction mechanism which PD-1 bears. As one aspect of this mechanism, PD-1 inhibits T cell activation in collaboration with its ligands PD-L1 and PD-L2. PD-1 (Programmed cell death-1) is a membrane protein expressed in activated T cells and B cells. Its ligands PD-L1 and PD-L2 are expressed in various cells such as antigen-presenting cells (monocytes, dendritic cells, etc.) and cancer cells. PD-1, PD-L1 and PD-L2 work as inhibitory factors which inhibit T cell activation. Certain types of cancer cells and virus-infected cells escape from host immune surveillance by expressing the ligands of PD-1 to thereby inhibit T cell activation.

As PD-1 signaling inhibitors, substances which specifically bind to PD-1, PD-L1 or PD-L2 may be given. Such substances include, but are not limited to, proteins, polypeptides, oligopeptides, nucleic acids (including natural-type and artificial nucleic acids), low molecular weight organic compounds, inorganic compounds, cell extracts, and extracts from animals, plants, soils or the like. These substances may be either natural or synthetic products. Preferable PD-1 signaling inhibitors are antibodies. More preferably, antibodies such as anti-PD-1 antibody, anti-PD-L1 antibody and anti-PD-L2 antibody may be given. Any type of antibody may be used as long as it is capable of inhibiting PD-1 signaling. The antibody may be any of polyclonal antibody, monoclonal antibody, chimeric antibody, single chain antibody, humanized antibody or human-type antibody. Methods for preparing such antibodies are known. The antibody may be derived from any organisms such as human, mouse, rat, rabbit, goat or guinea pig. As used herein, the term "antibody" is a concept encompassing antibodies of smaller molecular sizes such as Fab, F(ab)'₂, ScFv, Diabody, V_{H}, V_{L}, Sc(Fv)₂, Bispecific sc(Fv)₂, Minibody, scFv-Fc monomer or scFv-Fc dimer. As PD-1 signaling inhibitors, nivolumab (Bristol-Myers Squibb), pembrolizumab (Merck), cemiplimab (Regeneron/Sanofi), camrelizumab (Jiangsu Hengrui), sintilimab (Innovent Biologics/Lilly) and toripalimab (Shanghai Junshi Bioscience), all targeting PD-1, as well as atezolizumab (Genetech/Roche), durvalumab (AstraZeneca) and avelumab (Merck/Pfizer), all targeting PD-L1, have been approved in many countries across the world. Further, tislelizumab (BeiGene), dostarlimab (AnaptysBio/Tesaro), HLX-04 (Shanghai Henlius Biotech), AK-105 (Akeso Biopharma), spartalizumab (Novartis), BCD-100 (Biocad), MGA-012 (Incyte), genolimzumab (Genor Biopharma), HX-008 (Taizhou Hanzhong BioMedical), BAT-1306 (Bio-Thera Solutions/Sun Yat-sen University (SYSU)), AMP-224 (National Cancer Institute (NCI)), BI-754091 (Boehringer Ingelheim), sasanlimab (Pfizer), ABBV-181 (AbbVie), AB-122 (Arcus Biosciences), Sym-021 (Symphogen), MGD-013 (MacroGenics), GLS-010 (Harbin Gloria Pharmaceuticals/WuXi PharmaTech) and F-520 (Shandong New Time Pharmaceutical), all targeting PD-1, as well as envafolimab (Jiangsu Alphamab Biopharmaceuticals), bintrafusp alfa (Merck KGaA), SL-279252 (Shattuck Labs), FS-118 (F-star) and CA-170 (Aurigene/Curis), all targeting PD-L1 are now under clinical development. These PD-1 signaling inhibitors may be used.

The pharmaceutical composition of the present invention may be used as an anti-cancer agent, an anti-infective agent or a combination thereof.

When the pharmaceutical composition of the present invention is administered as an anticancer agent, target cancers or tumors includes, but are not limited to, leukemia, lymphoma (e.g., Hodgkin's disease, non-Hodgkin's lymphoma), multiple myeloma, brain tumors, breast cancer, endometrial cancer, cervical cancer, ovarian cancer, esophageal cancer, stomach cancer, appendix cancer, colon cancer, liver cancer, gallbladder cancer, bile duct cancer, pancreatic cancer, adrenal cancer, gastrointestinal stromal tumor, mesothelioma, head and neck cancer (such as laryngeal cancer), oral cancer (such as floor of mouth cancer), gingival cancer, tongue cancer, buccal mucosa cancer, salivary gland cancer, nasal sinus cancer (e.g., maxillary sinus cancer, frontal sinus cancer, ethmoid sinus cancer, sphenoid sinus cancer), thyroid cancer, renal cancer, lung cancer, osteosarcoma, prostate cancer, testicular tumor (testicular cancer), renal cell carcinoma, bladder cancer, rhabdomyosarcoma, skin cancer (e.g., basal cell cancer, squamous cell carcinoma, malignant melanoma, actinic keratosis, Bowen's disease, Paget's disease) and anal cancer.

When the pharmaceutical composition of the present invention is administered as an anti-infective agent, target infections include, but are not limited to, bacterial infections [various infections caused by Streptococcus (e.g., group A β hemolytic streptococcus, pneumococcus), Staphylococcus aureus (e.g., MSSA, MRSA), Staphylococcus epidermidis, Enterococcus, Listeria, Neisseria meningitis aureus, Neisseria gonorrhoeae, pathogenic Escherichia coli (e.g., 0157:H7), Klebsiella (Klebsiella pneumoniae), Proteus, Bordetella pertussis, Pseudomonas aeruginosa, Serratia, Citrobacter, Acinetobacter, Enterobacter, mycoplasma, Clostridium or the like; tuberculosis, cholera, plague, diphtheria, dysentery, scarlet fever, anthrax, syphilis, tetanus, leprosy, Legionella pneumonia (legionellosis), leptospirosis, Lyme disease, tularemia, Q fever, and the like], rickettsial infections (e.g., epidemic typhus, scrub typhus, Japanese spotted fever), chlamydial infections (e.g., trachoma, genital chlamydial infection, psittacosis), fungal infections (e.g., aspergillosis, candidiasis, cryptococcosis, trichophytosis, histoplasmosis, Pneumocystis pneumonia), parasitic protozoan infections (e.g., amoebic dysentery, malaria, toxoplasmosis, leishmaniasis, cryptosporidiosis), parasitic helminthic infections (e.g., echinococcosis, schistosomiasis japonica, filariasis, ascariasis, diphyllobothriasis latum), and viral infections [e.g., influenza, viral hepatitis, viral meningitis, acquired immune deficiency syndrome (AIDS), adult T-cell leukemia, Ebola hemorrhagic fever, yellow fever, cold syndrome, rabies, cytomegalovirus infection, severe acute respiratory syndrome (SARS), progressive multifocal leukoencephalopathy, chickenpox, herpes zoster, hand-foot-and-mouth disease, dengue, erythema infectiosum, infectious mononucleosis, smallpox, rubella, acute anterior poliomyelitis (polio), measles, pharyngoconjunctival fever (pool fever), Marburg hemorrhagic fever, hantavirus renal hemorrhagic fever, Lassa fever, mumps, West Nile fever, herpangina and chikungunya fever].

The pharmaceutical composition of the present invention comprises a substance capable of enhancing TCR signaling and is administered before, after or simultaneously with the administration of a PD-1 signaling inhibitor.

In the pharmaceutical composition of the present invention, a PD-1 signaling inhibitor and a substance capable of enhancing TCR signaling may be administered separately at different times or at the same time; if desired, they may be administered together simultaneously. When a PD-1 signaling inhibitor and a substance capable of enhancing TCR signaling are administered together simultaneously, it is convenient to prepare a combination drug containing the PD-1 signaling inhibitor and the substance capable of enhancing TCR signaling.

The pharmaceutical composition of the present invention is administered to subjects systemically or locally by an oral or parenteral route. The subjects include human beings or non-human animals.

PD-1 signaling inhibitors (e.g., anti-PD-1 antibody, anti-PD-L1 antibody or anti-PD-L2 antibody) may be dissolved in buffers such as PBS, physiological saline or sterile water, optionally filter- or otherwise sterilized before being administered to subjects by injection or infusion. To the solution of PD-1 signaling inhibitors, additives such as coloring agents, emulsifiers, suspending agents, surfactants, solubilizers, stabilizers, preservatives, antioxidants, buffers, isotonizing agents and the like may be added. As routes of administration, intravenous, intramuscular, intraperitoneal, subcutaneous or intradermal administration and the like may be selected.

The content of the PD-1 signaling inhibitor (e.g., anti-PD-1 antibody, anti-PD-L1 antibody or anti-PD-L2 antibody) in a preparation varies with the type of the preparation and is usually 1-100% by weight, preferably 50-100% by weight. Such a preparation may be formulated into a unit dosage form.

The dose and the number of times and frequency of administration of the PD-1 signaling inhibitor (e.g., anti-PD-1 antibody, anti-PD-L1 antibody or anti-PD-L2 antibody) may vary with the symptoms, age and body weight of the subject, the method of administration, dosage form and so on. For example, in terms of the amount of the active ingredient, 0.1-100 mg/kg body weight, preferably 1-10 mg/kg body weight, may usually be administered per adult at least once at a frequency that enables confirmation of the desired effect.

The substance capable of enhancing TCR signaling may be contained in a preparation comprising a PD-1 signaling inhibitor. Alternatively, the substance either alone or in admixture with an excipient or carrier may be formulated into tablets, capsules, powders, granules, liquids, syrups, aerosols, suppositories, injections or the like. The excipient or carrier may be of any type that is routinely used in the art and pharmaceutically acceptable, with their type and composition being appropriately changed. As a liquid carrier, for example, water, plant oil or the like may be used. As a solid carrier, saccharides such as lactose, sucrose or glucose, starches such as potato starch or corn starch, cellulose derivatives such as microcrystalline cellulose, and the like may be used. Lubricants such as magnesium stearate, binders such as gelatin or hydroxypropyl cellulose, and disintegrants such as carboxymethyl cellulose, and the like may be added. What is more, antioxidants, coloring agents, flavoring agents, preservatives, and the like may also be added.

The substance capable of enhancing TCR signaling may be administered via various routes such as oral, transnasal, rectal, transdermal, subcutaneous, intravenous or intramuscular route.

The content of the substance capable of enhancing TCR signaling varies with the type of the preparation and is usually 1-100% by weight, preferably 50-100% by weight. In the case of a liquid, for example, the content of the substance capable of enhancing TCR signaling in the preparation is preferably 1-100% by weight. In the case of a capsule, tablet, granule or powder, the content of the substance capable of enhancing TCR signaling in the preparation is usually 10-100% by weight, preferably 50-100% by weight, with the balance being the carrier. The preparation may be formulated into a unit dosage form.

The dose and the number of times and frequency of administration of the substance capable of enhancing TCR signaling may vary with the symptoms, age and body weight of the subject, the method of administration, dosage form and so on. For example, when the substance capable of enhancing TCR signaling is a CD45 inhibitor, usually approx. 0.005 µg (or µl) - 25000 mg (or ml)/kg body weight, preferably approx. 0.05 µg (or µl) - 12500 mg (or ml)/kg body weight, in terms of the amount of the active ingredient may be administered per adult at least once at a frequency that enables confirmation of the desired effect. When the substance capable of enhancing TCR signaling is a cell, usually from approx. 1 × 10⁶ cells/person to 1 × 10¹¹ cells/person, preferably from approx. 5 × 10⁶ cells/person to 2 × 10¹⁰ cells/person, may be administered per adult at least once at a frequency that enables confirmation of the desired effect.

The ratio (in mass) of PD-1 signaling inhibitor (e.g., anti-PD-1 antibody, anti-PD-L1 antibody or anti-PD-L2 antibody) to the substance capable of enhancing TCR signaling is suitably from 1:0.1 to 1:100, preferably from 1:1 to 1:50, when the substance capable of enhancing TCR signaling is a CD45 inhibitor. When the substance capable of enhancing TCR signaling is a cell, suitably from 1 × 10⁶ to 1 × 10¹⁰ cells, preferably from 5 × 10⁶ to 2 × 10⁹ cells may be used relative to 240 mg of the PD-1 signal inhibitor.

The present invention also provides a method of preventing and/or treating cancer, infection or a combination thereof, comprising administering to a subject a pharmaceutically effective amount of a substance capable of enhancing TCR signaling before, after or simultaneously with the administration of a PD-1 signaling inhibitor. Further, the present invention provides use of a substance capable of enhancing TCR signaling for treating cancer, infection or a combination thereof, wherein the substance capable of enhancing TCR signaling is administered before, after or simultaneously with the administration of a PD-1 signaling inhibitor. Still further, the present invention provides use of a substance capable of enhancing TCR signaling in a method of treating cancer, infection or a combination thereof, wherein the substance capable of enhancing TCR signaling is administered before, after or simultaneously with the administration of a PD-1 signaling inhibitor. The present invention also provides a substance capable of enhancing TCR signaling for use in preventing and/or treating cancer, infection or a combination thereof, said substance being administered before, after or simultaneously with the administration of a PD-1 signaling inhibitor.

The present invention provides a drug which enhances PD-1 signaling inhibitory activity, comprising a substance capable of enhancing TCR signaling. The present invention also provides a method of enhancing PD-1 signaling inhibitory activity, comprising administering to a subject a pharmaceutically effective amount of a substance capable of enhancing TCR signaling before, after or simultaneously with the administration of a PD-1 signaling inhibitor.

The drug of the present invention may be used in combination with a PD-1 signaling inhibitor or be formulated with a PD-1 signaling inhibitor. As regards the combined use of a PD-1 signaling inhibitor and a substance capable of enhancing TCR signaling and as for the way to formulate the inhibitor and the substance into a combination drug, reference should be made to the foregoing description. The drug of the present invention is applicable not only as a pharmaceutical drug but also as an experimental reagent.

Furthermore, the present invention provides a TCR signaling enhancer comprising a CD45 inhibitor and/or a cell. The present invention also provides a method of enhancing TCR signaling, comprising administering to a subject a pharmaceutically effective amount of a CD45 inhibitor and/or a cell. As regards CD45 inhibitors and cells, reference should be made to the foregoing description. The enhancer of the present invention is applicable not only as a pharmaceutical drug but also as an experimental reagent. Enhancement of TCR signaling leads to improvement of the antitumor effect of PD-1 signaling inhibitor. In addition to that, other effects such as augmentation of therapeutic effect against infections can also be obtained.

### Examples

Hereinbelow, the present invention will be described in more detail with reference to the following Examples.

### [Example 1]

### [Abstract]

CD8⁺ T cells play a central role in anti-tumor immune responses that kill cancer cells directly. In aged individuals, CD8⁺ T-cell immunity is strongly suppressed, which is associated with cancer and other age-related diseases. This study investigated the role of T-cell function in age-related unresponsiveness to PD-1 blockade cancer therapy. The present inventors found inefficient generation of CD44^{low}CD62L^{low} CD8⁺ T-cell subset (P4) in draining lymph nodes of tumor-bearing aged mice. *In vitro* stimulation of naive CD8⁺ T cells first generated P4 cells, followed by differentiation into effector/memory T cells. In the P4 cells, expression of a unique set of genes of enzymes involved in one-carbon (1C) metabolism was specifically high; these genes are critical to antigen-specific T-cell activation and mitochondrial function. Consistent with this finding, 1C-metabolism-related gene expression and mitochondrial respiration were downregulated in aged CD8⁺ T cells compared with young CD8⁺ T cells. In aged ovalbumin (OVA)-specific T-cell receptor (TCR) transgenic mice, ZAP-70 was not activated, even after inoculation of OVA-expressing tumor cells. This attenuation of TCR signaling appeared to be due to elevated expression of CD45RB phosphatase in aged CD8⁺ T cells. Surprisingly, strong stimulation by injection of non-self cells into aged PD-1 deficient mice restored normal levels of CD45RB, and ameliorated the emergence of P4 cells and 1C metabolic enzyme expression in CD8⁺ T cells, as well as anti-tumor activity. These findings suggest that impaired induction of the P4 subset may be responsible for the age-related resistance to PD-1 blockade, which can be rescued by strong TCR stimulation.

### [Significance Statement]

Although aging is known to suppress anti-tumor immunity, the precise underlying mechanism remains largely unknown. Here, the present inventors have found that the inefficient generation of CD44^{low}CD62L^{low} CD8⁺ T-cell subset (P4), pre-effector-like T cells, could explain the resistance to PD-1 blockade anti-tumor therapy in aged mice. Elevated expression of CD45RB in aged naive CD8⁺ T cells appears to inhibit TCR signaling, resulting in fewer P4 cells, a subset with high expression of 1C metabolic genes. This decrease in P4 cells and anti-tumor activity were rescued by strong immunogenic stimulation by non-self cells. These findings provide valuable insights into the mechanism underlying age-induced suppression of anti-tumor immunity, which may provide a basis for the development of therapeutic strategies for elderly cancer patients.

### [Introduction]

Aging affects numerous physiological functions, resulting in the onset of a variety of diseases. Cancer risk increases with age, as mutations accumulate in the genome and immune surveillance against cancer cells gradually declines (1, 2). Immunotherapy that reactivates tolerized immune function has emerged as an effective strategy for treating cancer. Among the array of cancer immune therapeutics, antibodies that block the PD-1/PD-L1 pathway have yielded highly promising results in patients with a broad spectrum of cancers (3-6). However, clinical studies have shown that many cancer patients are unresponsive to PD-1 blockade therapy (7, 8). The efficacy of such therapy is impaired in aged-mouse models, with similar attenuation to that observed in some clinical reports (9-12).

Immune senescence results from quantitative and/or qualitative changes in immune cells. One of the major quantitative changes in T cells is the decline of the TCR repertoire diversity due to thymic involution, reducing the output of naive cells into the periphery (13-15). In addition, peripheral T cells in aged individuals accumulate functional defects such as impaired TCR signaling, diminished differentiation capacity to effector and memory cells, and reduced cytokine production (16, 17). In fact, age-associated changes strongly affect the frequency of well-defined CD8⁺ T-cell subsets (18-20). CD44 and CD62L (L-selectin) surface markers are used to define 3 major subsets of CD8⁺ T cells in mice: naive (also called P1; CD44^{low}CD62L^{high}), central memory (P2; CD44^{high}CD62L^{high}), and effector/memory (P3; CD44^{high}CD62L^{low}). The remaining CD44^{low}CD62L^{low} (P4) CD8⁺ T-cell subset is a very minor population in naive mice. Naive T cells differentiate into effector T cells by antigenic stimulation (14, 21). Some effector cells differentiate into memory cells, which are capable of differentiating into effector cells quickly in response to the same antigenic stimulation. In aged humans and animals, T-cell maintenance requires self-antigen-dependent proliferation (homeostatic proliferation) because of the decline in T-cell output from the thymus (22, 23). In aged individuals, this homeostatic proliferation gradually boosts naive T-cell differentiation, resulting in an increased frequency of differentiated T-cell subsets including effector and memory cells.

T-cell proliferation and differentiation are promoted by T-cell receptor (TCR) signaling cascades. The phosphorylation and dephosphorylation of TCR signaling molecules affect signaling complex formation and the propagation of TCR signals. CD45 is a transmembrane phosphatase that plays a central role in the modulation of TCR signaling by regulating the level of tyrosine phosphorylation in lymphocyte protein kinase (Lck). Lck serves as an activator of TCRζ, ζ-chain-associated protein (ZAP)-70 (these are downstream factors of TCR signaling) and the linker of activated T cells (LAT) (24, 25). In young naive CD8⁺ T cells, high CD45 expression inhibits TCR signal transduction through dephosphorylation of ZAP-70 and PLCy (26). While aging is known to alter TCR signaling, the molecular mechanisms underlying these changes remain largely unclear (27, 28).

In activated and proliferating cells, one-carbon (1C) metabolism is generally upregulated, supporting their survival and differentiation through promoting biosynthesis of purine and thymidine, amino acid homeostasis, epigenetic maintenance, and redox defense (29, 30). Recent reports indicate that 1C metabolism is one of the most strongly induced metabolic pathways during early CD4⁺ or CD8⁺ T-cell activation (31, 32). After T-cell activation, upregulation of the 1C metabolic network increases the processing of serine to provide *de novo* nucleotide biosynthesis from one-carbon units, which is required for antigen-specific T-cell proliferation, differentiation, and effector functions. The 1C metabolic pathways also contribute to energy production through the regulation of mitochondrial protein synthesis by mitochondrial tRNA methylation (33, 34). Mitochondrial function and 1C metabolism are lower in aged than in young CD4⁺ T cells (35), suggesting a possible link between T-cell aging and the inactivation of 1C metabolism.

To elucidate the mechanism underlying age-related attenuation of the anti-tumor effect of PD-1 blockade, the present inventors compared CD8⁺ T cells from young and aged PD-1 knock-out (KO) or wild type (WT) mice with or without tumor inoculation. In this study, the present inventors have found that resistance to PD-1 deficiency or blockade anti-tumor therapy in aged mice is due to inefficient generation of P4 subset, revealing that P4 subset is an intermediate between the naive and effector subsets and highly expresses genes involved in 1C metabolism. This inhibition of P4 subunit in aged mice is due to TCR signaling inhibition through elevated expression of CD45RB and can be rescued by strong immune stimulation. These findings provide novel insights into the mechanisms involved in age-dependent changes in CD8⁺ T-cell subsets that contribute to metabolic alterations and anti-tumor activity.

### [Results]

Impaired induction of CD44^{low}CD62^{low} (P4) CD8⁺ T cells in aged mice.

Aged PD-1 KO mice became permissive to tumor growth while young PD-1 KO mice were strongly resistant to tumor growth (Fig. 1A and B). The present inventors also observed that aged WT mice were resistant to PD-1 blockade tumor therapy (Fig. 6A and B). Comparative analysis of CD8⁺ T-cell subsets in cells of peripheral lymph nodes (PLNs) and draining lymph nodes (DLNs) from PD-1 KO mice with or without tumors showed that the percentage of the minor CD8⁺ T-cell subset P4 was substantially increased by tumor inoculation in young mice but not in aged mice (Fig. 1C and D). Changes in other CD8⁺ T-cell subsets in response to tumor inoculation were smaller than changes in the P4 subset in both young and aged PD-1 KO mice (Fig. 1C and D). Similar results were also obtained in WT mice (Fig. 6C and D). These results suggest that the P4 subset is inducible during tumor rejection and may be necessary for the anti-tumor activity observed when PD-1 is blocked or deficient.

P4 cells are intermediates between P1 and P3 cells and have the potential of anti-tumor activity.

To examine whether the attenuated P4 cell induction contributes to the resistance to PD-1 blockade therapy in aged mice, the present inventors characterized the functional properties of the CD8⁺ P4 subset. The present inventors first investigated the anti-tumor activity of P4 cells by injecting isolated P4 cells into young tumor-bearing CD8 KO mice. To avoid TCR repertoire bias, the present inventors used young TCR-transgenic OT-1 mice, whose CD8⁺ T cells express a unique TCR specifically responsive to ovalbumin (OVA) peptide. P3 and P4 subsets of CD8⁺ T cells were induced by injection of OVA-expressing MC38 tumor cells (MC38-OVA) into the tail vein of OT-1 mice, and isolated from the spleen 5 days later. The same number of P3 or P4 cells was adoptively transferred into MC38-OVA-injected young CD8 KO mice. MC38-OVA growth was strongly suppressed in the mice which received transfer of either P3 or P4 cells (Fig. 2A). Notably, all of the transferred P4 cells had differentiated into P3 cells in the peripheral blood 6 days after the transfer (Fig. 2B). These results suggest that P4 cells have the potential to reject tumors by differentiating into P3 cells.

Although P1 and P2 cells are known to differentiate into P3 cells during activation, P4 cells have not been well characterized because of their scarcity. To determine from which subset P4 cells originate and to which subset they differentiate, P1, P2, and P4 cells were isolated from young PD-1 KO mice and stimulated *in vitro* with anti-CD3/CD28 antibodies and IL-2 (Fig. 2C). In agreement with the results of the *in vivo* study, isolated P4 cells exclusively differentiated to P3 cells under these conditions (Fig. 2D). Next, the present inventors investigated whether P4 cells are generated from P1 or P2 cells. As shown in Fig. 2E and 2F, P4 cells were generated from P1 cells but not from P2 cells two days after *in vitro* stimulation. These results indicate that stimulated naive P1 CD8⁺ T cells give rise to P4 cells that further differentiate into P3 cells. Since *in vivo* studies indicate that P4 cell induction is defective in aged mice (Fig. 1C and D), the present inventors tested the potential of aged P1 cells to generate P4 cells *in vitro.* Surprisingly, P4 and P3 cells were induced from aged P1 cells as efficiently as from young P1 cells following culture with anti-CD3/CD28 antibodies and IL-2 (Fig. 2G). These findings suggest that P1 cells from aged mice can be directed to differentiate into P4 cells by a strong *in vitro* TCR stimulant such as anti-CD3/CD28 antibody but not by normal antigenic stimulation.

One-carbon-metabolism-related genes are highly expressed in P4 cells.

To understand the role of P4 cells in anti-tumor activity in aged mice, the present inventors compared the gene expression profiles among CD8⁺ T-cell subsets (P1, P2, P3, and P4) in young PD-1 KO mice. Hierarchical clustering of global gene expression showed that each subset of CD8⁺ T cells has a distinct gene expression profile (Fig. 3A). Genes expressed in activated T cells (such as Ctla4, Prdm1, and Il2rb) were more highly expressed in P4 cells than in P1 cells (Fig. 3B). In contrast, genes expressed in differentiated T cells (such as Il7r, Ifng, and Tbx21) were expressed at lower levels in P4 cells than in P2 or P3 cells (Fig. 3B). These results suggest that P4 cells are in an activated but pre-mature state and distinct from more differentiated subsets of CD8⁺ T cells such as P2 and P3 cells, supporting the present inventors' hypothesis that P4 cells are pre-effector-like cells derived from naive P1 cells.

Next, gene ontology enrichment analysis was applied to determine which biological processes were upregulated in these CD8⁺ T-cell subsets. In P4 cells, the most significantly upregulated processes were related to 1C metabolic pathways that mediate the interconversion of serine, glycine, and folate derivatives (Fig. 3C and D). The expression of 1C-metabolism-related genes was highest in P4 cells, as shown by microarray analysis and qPCR (Fig. 3E and F). These results indicate that 1C metabolic pathways are selectively upregulated in P4 cells during CD8⁺ T-cell differentiation.

Tumor inoculation increased the expression of 1C-metabolism-related genes in the total population of CD8⁺ T cells in young WT and PD-1 KO mice (Fig. 3G), which could be explained by the generation of P4 cells (Fig. 1C and Fig. 6C). Consistent with the higher efficiency of P4 cell induction in PD-1 KO mice (Fig. 1C and Fig. 6C), the CD8⁺ T cells of young PD-1 KO mice expressed higher levels of most of the 1C metabolism-related genes than those cells of young WT mice in response to MC38 tumor cell inoculation (Fig. 3G). In contrast, expression of these genes in the total CD8⁺ T cells from aged mice did not markedly change upon tumor inoculation, regardless of mouse genotype.

Elevated mitochondrial function is critically involved in CD8⁺ T-cell activation during immune response (38, 39). Since P4 cells exhibited high expression of Shmt2, a regulator of mitochondrial tRNA modification, the present inventors suspect that upregulation of 1C metabolism may increase anti-tumor activity in part through enhancing mitochondrial function. To examine mitochondrial activity in young and aged mice, the present inventors compared the oxygen consumption rate (OCR) in total CD8⁺ T cells between tumor-bearing young and aged mice. Aged CD8⁺ T cells from WT or PD-1 KO mice showed clear abnormality in the OCR, with substantial loss of basal respiration and spare respiratory capacity (Fig. 3H and I). Taken together, these results suggest that defective P4 cell induction in aged mice may lead to reduced mitochondrial function in the CD8⁺ T-cell population, possibly through inefficient upregulation of 1C metabolic pathways.

### Elevated CD45RB expression in aged mice

Since P4 cells were shown to be generated from P1 cells by *in vitro* stimulation, the present inventors suspected that the lower level of P4 cell induction upon tumor inoculation observed in aged mice could be caused by inefficient transduction of TCR signals in aged P1 cells. To examine the relationship between TCR signaling and P4 cell generation, the present inventors injected MC38-OVA into OT-1 mice. The percentage of P4 cells in peripheral lymph node (PLN) cells was markedly increased by this infusion in young but not aged OT-1 mice (Fig. 4A and B). To evaluate TCR signal transduction, the present inventors investigated the phosphorylation of ZAP-70, which is critical to TCR signaling (40), in total CD8⁺ T cells from young and aged OT-1 mice after MC38-OVAinjection. MC38-OVAinjection increased the percentage of CD8⁺ T cells expressing phosphorylated ZAP-70 in young but not aged OT-1 mice (Fig. 4C), suggesting that activation of TCR signaling by antigenic stimulation is inhibited in aged OT-1 mice. Notably, no significant difference was observed in the intensity of the OVA-specific MHC tetramer (Fig. 7A and B), indicating that the TCR expression levels and/or avidity of young and aged OT-1 T cells were similar. Therefore, the inhibition of antigen-stimulated activation of TCR signaling observed in aged OT-1 mice must involve a mechanism other than TCR and MHC/antigen interaction.

Because the response of naive CD8⁺ T cells to TCR stimulation is weaker in cells with a high density of cell-surface CD45 (26), the present inventors analyzed the expression level of CD45RB, a major CD45 isoform in mouse naive and memory T cells. CD45RB expression in total CD8⁺ T cells was remarkably higher in aged than in young OT-1 mice (Fig. 4D). The effect of aging on CD45RB expression was greater in P1 and P4 cells than in P2 or P3 cells (Fig. 4D). Similarly, the greatest increase in CD45RB expression was observed in P1 cells of aged WT and PD-1 KO mice (Fig. 8A and B), suggesting that the inhibition of P4 cell induction may possibly result from increased CD45RB expression in P1 cells. To examine whether CD45RB expression suppresses CD8⁺ T-cell activation by TCR signal transduction, the present inventors evaluated the phosphorylation of ZAP-70 in OT-I CD8⁺ T cells after treatment with a CD45-specific inhibitor. Consistent with the *in vivo* results, the percentage of p-ZAP-70⁺ CD8⁺ T cells was increased by co-culture with MC38-OVA cells in young but not aged CD8⁺ T cells (Fig. 4E). Under these conditions, the addition of a CD45 inhibitor increased p-ZAP-70⁺ cell induction in aged as well as young CD8⁺ T cells. These data indicate that age-related inefficiency in tumor rejection is likely due to increased expression of CD45RB in aged CD8⁺ T cells.

Xenogeneic cell treatment increases P4 cell induction and ameliorates the anti-tumor capacity of CD8⁺ T cells in aged mice.

Since the suppression of P1 to P4 transition in aged mice can be recovered by strong *in vivo* stimulation as shown above, the present inventors investigated whether strong stimulation using xenogeneic cells (human Burkitt lymphoma, Daudi cells) could recover P4 CD8⁺ T-cell induction in aged PD-1 KO mice (Fig. 5A). Ten days after Daudi-cell injection into the tail vein (day 0: before MC38 tumor inoculation), the percentage of P4 cells was markedly increased in PLNs of both young and aged PD-1 KO mice, but the percentages of P1-P3 cells were not significantly increased (Fig. 5B). As expected, the expression levels of genes involved in 1C metabolism in CD8⁺ T cells from aged PD-1 KO mice were increased by the Daudi-cell injection (Fig. 5C). Consistent with these findings, Daudi-cell injection reduced CD45RB expression in the P1 cells of aged PD-1 KO mice (Fig. 5D).

Daudi-cell injection dramatically inhibited MC38 tumor growth in aged PD-1 KO mice and increased their survival (Fig. 5E and F). Age-related unresponsiveness to anti-PD-L1 antibody in WT mice was also recovered by Daudi-cell injection (Fig. 9A-E). On day 6 after tumor inoculation, the percentage of p-ZAP-70 positive cells was augmented in Daudi-cell-injected PD-1 KO mice (Fig. 5G), suggesting that xenogeneic stimulation augments TCR signaling against tumor antigens. The present inventors further investigated whether Daudi-cell treatment increases the proliferation of tumor antigen-reactive CD8⁺ T cells. MC38-OVA cells were inoculated into PD-1 KO mice 10 days after Daudi-cell injection and the emergence of OVA tetramer⁺ CD8⁺ T cells was monitored. The percentage of OVA tetramer⁺ CD8⁺ T-cells was elevated in DLNs and tumor sites in aged PD-1 KO mice by the injection of Daudi cells (Fig. 5H and I), indicating that this treatment resulted in increase in antigen-specific CD8⁺ T-cell induction. Given that pre-stimulation with Daudi cells boosts P4 cell induction and 1C metabolism in CD8⁺ T cells of aged mice, it is reasonable that Daudi-cell injection increased both the basal respiration and spare respiratory capacity of aged CD8⁺ T cells (Fig. 10A-C). Injection of splenocytes from Balb/c mice as alloantigen also increased the anti-tumor effects of PD-1 blockade therapy in aged WT mice (Fig. 11A-D). These data suggest that strong immune stimulation by xeno- or allo-geneic cell treatment rescues the compromised anti-tumor response of CD8⁺ T cells in aged mice and improves the anti-tumor effect of PD-1 deficiency or PD-1 blockade therapy. Taken together, these findings show that strong immune stimulation by non-self cells can rescue the insufficient anti-tumor response in aged mice through the recovery of TCR signal transduction and the induction of P4 subset, which enhances the efficacy of PD-1 deficiency and blockade therapy.

### [Discussion]

Aged individuals and animals are known to be insensitive to immunotherapy because of age-related suppression of CD8⁺ T-cell responses (9-11). The mechanism underlying the suppression of CD8⁺ T-cell response to tumor antigen is largely unknown. The present inventors found that aged tumor-bearing mice had significantly fewer P4 cells due to weakened TCR signaling caused by increase in CD45RB expression. To date, this P4 subset has been poorly characterized; P4 subset differentiates from naive P1 subset and further matures into effector/memory subset (P3) critical to anti-tumor activity (42, 43). P4 subset is unique in its higher expression of 1C-metabolism-related genes, which are associated with the growth and survival of proliferating cells (29, 32, 44, and 45). These aging-induced inhibitions in CD8⁺ T-cell differentiation and the efficacy of PD-1 blockade therapy are rescued by strong immunogenic stimulation using non-self cells.

Age-associated deviations in the naive T-cell repertoire have been proposed as the main reason for the suppression of T-cell responses in aged individuals (46, 47). However, the present inventors found that OT-1 transgenic mice, in which all CD8⁺ T cells carry OVA-specific TCR, became insensitive to specific antigens on tumor cells with aging. Our study further revealed that strong TCR stimulation *in vitro* or with xenogeneic cell treatment *in vivo* recovered P4 cell generation. In a similar line, an impaired anti-tumor response observed in aged OT-II CD4⁺ T cells, which express an OVA-specific TCR, is caused by impaired Th1 differentiation resulting from the IL-6-enriched environment that comes with aging (48). These results indicate that repertoire limitation may not be the major cause of the suppression of T-cell responses in aged animals.

Previous studies report that TCR signaling becomes suppressed with aging, leading to impaired T-cell responses (49, 50). The increase in the level of p-ZAP-70 by T-cell activation is smaller in aged humans and mice (51, 52). Consistent with these reports, the present inventors also found that ZAP-70 phosphorylation was lower in tumor-inoculated aged mice. Our data showed that this change was caused by an increase in CD45RB expression in aged P1 cells. CD45 negatively regulates TCR signaling by dephosphorylating tyrosine 394 of Lck (a tyrosine kinase), leading to suppression of ZAP-70 activation (53). Indeed, the induction of p-ZAP-70⁺ CD8⁺ T cells was recovered by treatment with a CD45 inhibitor. These results strongly support the hypothesis that suppression of CD8⁺ T-cell responses in aged animals is caused by deficient TCR signaling in naive T cells via increased expression of CD45RB.

Alarge number of T cells are activated by allo- or xeno-geneic MHC molecules, either directly (e.g., donor MHC-presenting donor peptides) or indirectly (e.g., recipient MHC-presenting donor peptides) (54-56). The present inventors speculate that P4 cell recovery in aged mice is mediated by TCR recognition of non-self-antigens. Since TCR recognizes a broad range of different peptides in nature (57, 58), the P4 cells or the pre-activated P1 cells recovered by non-self-antigens cross react with tumor antigens, leading to an increase in anti-tumor responses. Our data also suggest that xenogeneic cell treatment retains a potential to reduce CD45RB expression in CD8⁺ P1 cells of aged mice. Further study will be required to elucidate the molecular mechanism by which CD45RB expression is regulated by aging and non-self cells.

### [Materials and Methods]

### Mice and Cells

All mice were maintained under specific pathogen (that would hinder experiments)-free conditions at the Institute of Laboratory Animals, Graduate School of Medicine, Kyoto University or RIKEN Center for Integrative Medical Sciences, and used according to appropriate experimental guidelines. PD-1^{-/-} (PD-1 KO) mice were prepared by homologous recombination in the laboratory of the present inventors (59). C57BL/6 WT mice were purchased from Charles River Laboratories Japan. OT-1 TCR-transgenic and CD8^{-/-} (CD8 KO) mice were purchased from The Jackson Laboratory (Bar Harbor, Maine USA) (originally deposited by M. B. Bevan at Washington Medical Center, or T. Mak at University of Toronto). The murine colon adenocarcinoma (MC38) cell line was kindly provided by J. P. Allison, Memorial Sloan-Kettering Cancer (New York, NY), and the MC38 cell line expressing ovalbumin (OVA) (MC38-OVA) was prepared by transducing MC38 cells with pMXs-based OVA-F2A-EGFP retrovirus. Cells were maintained in RPMI 1640 (Gibco; 11875-093) supplemented with 10% heat-inactivated fetal bovine serum (FBS) and 1% penicillin-streptomycin (Nacalai Tesque; 26253-84) under mycobacterial infection-free conditions.

### Mouse Therapy Models

MC38 cells (5 × 10⁵ or 2 × 10⁶) were intradermally (i.d.) injected into the right flank of mice (day 0). Five days after MC38-inoculation (day 5), anti-PD-L1 antibody (clone 1-111A.4; prepared and stored at the laboratory of the Department of Immunology and Genomic Medicine, Kyoto University Graduate School of Medicine; Immunology Letters 84(2002) 57-62) (6 mg/kg) was intraperitoneally injected into the mice. Administration of this anti-PD-L1 antibody was repeated 3 times at an interval of 6 days (on days 5, 11, and 17). An isotype of anti-PD-L1 antibody (Rat IgG2a) was used as a control. Tumor growth was monitored by measuring the tumor size using calipers, and the volume calculated using the following formula: π (length × breadth × height)/6. Non-self cell treatment was performed 10 days before tumor cell inoculation by injection into the tail vein of human Burkitt's lymphoma Daudi cells or mitomycin C (MMC) treated-splenocytes (2 × 10⁶ cells/mouse) from BALB/c mice or C57BL/6N mice as a control.

### Primary Cell Isolation for Analysis and Culture

Peripheral or draining lymph node (DLN) cells and tumor-infiltrating lymphocytes were isolated as previously described (39). For splenocyte analysis, spleens were loosened quickly and suspended in ammonium chloride potassium buffer to lyse red blood cells. The splenocytes were washed and resuspended in RPMI 1640 medium (T-cell medium) supplemented with 10% fetal bovine serum, L-glutamine, 55 µM 2-mercaptoethanol, penicillin-streptomycin, 1 mM sodium pyruvate (Gibco), and 1% MEM non-essential amino acid solution (Gibco). The present inventors further isolated subsets of CD8⁺ T cells using CD8a (Ly-2) microbeads (Miltenyi Biotec; 130-117-044) and FACSAria (BD Biosciences). For peripheral blood cell analysis, collected peripheral blood cells were treated with ammonium chloride potassium buffer and washed with T-cell medium. Purified cells were stimulated with anti-CD3/CD28 Dynabeads (Gibco) and recombinant human IL-2 (20 U/mL; PeproTech) in T-cell medium. To stimulate CD8⁺ T cells using MC38-OVA cells, CD8⁺ T cells isolated from lymph nodes of OT-1 mice were co-cultured with the MMC-treated MC38-OVA cells in T-cell medium. One day after stimulation, the cells were treated with 0.05 µM CD45 inhibitor VI (EDM Millipore) for 1 hour.

### Flow Cytometry analysis

The following monoclonal antibodies (mAbs) were used for analysis: CD3 (clone 145-2C11) and CD8 (clone 53-6.7) phosphor-ZAP70/Syk (clone n3kobu5) (Invitrogen) ; CD44 (clone IM7), and CD62L (clone MEL-14) (TONBO biosciences or BioLegend); CD8 (clone KT15, MBL Life Science); and CD45RB (clone 16A, BD Pharmingen). H-2Kb Negative (SIY) Tetramer-SIYRYYGL-APC (TS-5001-2C) and H-2Kb OVA Tetramer-SIINFEKL-APC (TS-M008-2) were obtained from MBL Life Science. Flow cytometry experiments were performed using FACSCant II (BD Biosciences) and analyzed using FlowJo software (FlowJo LLC).

### CD8 KO and OT-1 mouse models

To induce P3 and P4 cells, MC38-OVA cells (1-2 × 10⁶) were i.v. injected into the talk vein of OT-1 mice. After 5 days, P3 and P4 cells were isolated from those mice. Each of the isolated CD8⁺ T-cell subset was injected into the tail vein of CD8 KO mice that received intradermal injection of 2 × 10⁵ MC38-OVA cells 5 days ago. Peripheral blood was obtained 6 days after the inoculation to analyze inoculated cell differentiation.

### Microarray analysis

Total RNA was extracted from total CD8⁺ T cells pooled from 3 to 6 mice (per group) or from the CD8⁺ T-cell subset pooled from 9 mice (per group) using Nucleospin RNA (Macherey-Nagel) according to the manufacture's protocols. Microarray analysis was performed by Macrogen Inc. using the Mouse 8 × 60K v2 Microarray. The data were deposited in the GEO repository (http://www.ncbi.nlm.nih.gov/geo/) under the accession numbers GSE161659 and GSE161660. Gene ontology enrichment analysis was performed using the Database for Annotation, Visualization, and Integrated Discovery (DAVID; http://david.abcc.ncifcrf.gov/). Differentially expressed genes were visualized using R 3.1.2 or RStudio version 1.1.383.

### Real-time PCR

Total RNAwas isolated from cells using Nucleospin RNA (Macherey-Nagel) and used for cDNA synthesis using RevatraAce reverse transcriptase (Toyobo) and random primers according to the manufacturer's instructions. Real-time PCR was performed to amplify the indicated mRNAs using the Applied Biosystems 7500 Fast Real-Time PCR system (ABI) and PoweUp SYBRGreen Master Mix (Applied Biosystems). The expression level of each gene was normalized to the mRNA level of *β-actin.* The primer sequences were as follows:
*Shmt1* forward (Fw), 5'-CCAGAGTGCTGTGGCAACTC-3' (SEQ ID NO: 1);
*Shmt1* reverse (Rv), 5'-GCAAACACAGGCTGTTCCTG-3' (SEQ ID NO: 2);
*Shmt2* Fw, 5'-GACAGTTGAGGACACCTGGC-3' (SEQ ID NO: 3);
*Shmt2* Rv, 5'-CCAGAGAGGAGTGACATCTC-3' (SEQ ID NO: 4);
*Phgdh* Fw, 5'-TGGCCTCGGCAGAATTGGAAG-3' (SEQ ID NO: 5);
*Phgdh* Rv, 5'-TGTCATTCAGCAAGCCTGTGGT-3' (SEQ ID NO: 6);
*Psat1* Fw, 5'-GATGAACATCCCATTTCGCATTGG-3' (SEQ ID NO: 7);
*Psat1* Rv, 5'-GCGTTATACAGAGAGGCACGAATG-3' (SEQ ID NO: 8);
*β-actin* Fw, 5'-TAAGGCCAACCGTGAAAG-3' (SEQ ID NO: 9); and
*β-actin* Rv, 5'-GAGGCATACAGGGACAGCAC-3' (SEQ ID NO: 10).

### Oxygen consumption rate (OCR) analysis

OCR assay was performed as described previously (39), with minor modifications. OCR of CD8⁺ T cells isolated from DLNs was measured on an XFe96 Extracellular Flux analyzer (Agilent Technologies) using the Seahorse XFe96 Extracellular Flux assay kit and Seahorse XF Cell Mito Stress test kit (Agilent Technologies). CD8⁺ T cells (3 × 10⁵ per well) were seeded in Cell-Tak^{™} (Corning)-coated XFe96 plates. The spare respiratory capacity was calculated from the OCR graph as described previously (60). The basal respiration was calculated by subtracting the non-mitochondrial respiration (the value after rotenone/antimycin A addition from the value before oligomycin addition) (61).

### Statistical Analysis

Data were analyzed using Prism 7 (Graph pad), and the results are presented as the mean ± SEM. Comparisons of two groups were analyzed using unpaired two-tailed Student's t-test. When comparing more than two groups, statistical significance was analyzed using one-way analysis of variance (ANOVA) for multiple groups, followed by Tukey's post hoc test to analyze differences. Survival rates were evaluated using the Kaplan-Meier method, and statistical significance was determined by the log rank test.

### Data Availability

All sequencing data are registered at the NCBI GEO under accession ID numbers GSE161659 and GSE161660.

### References

1. T. Finkel, M. Serrano, M. A. Blasco, The common biology of cancer and ageing. Nature 448, 767-774 (2007).
2. K. Tomihara, T. J. Curiel, B. Zhang, Optimization of immunotherapy in elderly cancer patients. Crit Rev Oncog 18, 573-583 (2013).
3. S. L. Topalian et al., Safety, activity, and immune correlates of anti-PD-1 antibody in cancer. N Engl J Med 366, 2443-2454 (2012).
4. A. Ribas et al., Association of Pembrolizumab With Tumor Response and Survival Among Patients With Advanced Melanoma. JAMA 315, 1600-1609 (2016).
5. M. Reck et al., Pembrolizumab versus Chemotherapy for PD-L1-Positive Non-Small-Cell Lung Cancer. N Engl J Med 375, 1823-1833 (2016).
6. P. S. Chowdhury, K. Chamoto, T. Honjo, Combination therapy strategies for improving PD-1 blockade efficacy: a new era in cancer immunotherapy. J Intern Med 283, 110-120 (2018).
7. W. Zou, J. D. Wolchok, L. Chen, PD-L1 (B7-H1) and PD-1 pathway blockade for cancer therapy: Mechanisms, response biomarkers, and combinations. Sci Transl Med 8, 328rv324 (2016).
8. A. L. Shergold, R. Millar, R. J. B. Nibbs, Understanding and overcoming the resistance of cancer to PD-1/PD-L1 blockade. Pharmacol Res 145, 104258 (2019).
9. J. Brahmer et al., Nivolumab versus Docetaxel in Advanced Squamous-Cell Non-Small-Cell Lung Cancer. N Engl J Med 373, 123-135 (2015).
10. C. Helissey, C. Vicier, S. Champiat, The development of immunotherapy in older adults: New treatments, new toxicities? J Geriatr Oncol 7, 325-333 (2016).
11. A. Padron et al., Age effects of distinct immune checkpoint blockade treatments in a mouse melanoma model. Exp Gerontol 105, 146-154 (2018).
12. J. Sceneay et al., Interferon Signaling Is Diminished with Age and Is Associated with Immune Checkpoint Blockade Efficacy in Triple-Negative Breast Cancer. Cancer Discov 9, 1208-1227 (2019).
13. K. Naylor et al., The influence of age on T cell generation and TCR diversity. J Immunol 174, 7446-7452 (2005).
14. M. Ahmed et al., Clonal expansions and loss of receptor diversity in the naive CD8 T cell repertoire of aged mice. J Immunol 182, 784-792 (2009).
15. Q. Qi et al., Diversity and clonal selection in the human T-cell repertoire. Proc Natl Acad Sci U S A 111, 13139-13144 (2014).
16. J. Nikolich-Zugich, G. Li, J. L. Uhrlaub, K. R. Renkema, M. J. Smithey, Age-related changes in CD8 T cell homeostasis and immunity to infection. Semin Immunol 24, 356-364 (2012).
17. M. T. Ventura, M. Casciaro, S. Gangemi, R. Buquicchio, Immunosenescence in aging: between immune cells depletion and cytokines up-regulation. Clin Mol Allergy 15, 21 (2017).
18. E. J. Wherry, R. Ahmed, Memory CD8 T-cell differentiation during viral infection. J Virol 78, 5535-5545 (2004).
19. J. C. Nolz, G. R. Starbeck-Miller, J. T. Harty, Naive, effector and memory CD8 T-cell trafficking: parallels and distinctions. Immunotherapy 3, 1223-1233 (2011).
20. J. Nikolich-Zugich, The twilight of immunity: emerging concepts in aging of the immune system. Nat Immunol 19, 10-19 (2018).
21. B. Youngblood, J. S. Hale, R. Ahmed, T-cell memory differentiation: insights from transcriptional signatures and epigenetics. Immunology 139, 277-284 (2013).
22. D. Sauce et al., Lymphopenia-driven homeostatic regulation of naive T cells in elderly and thymectomized young adults. J Immunol 189, 5541-5548 (2012).
23. N. Minato, M. Hattori, Y. Hamazaki, Physiology and pathology of T-cell aging. Int Immunol 32, 223-231 (2020).
24. J. Rossy, D. J. Williamson, K. Gaus, How does the kinase Lck phosphorylate the T cell receptor? Spatial organization as a regulatory mechanism. Front Immunol 3, 167 (2012).
25. R. J. Brownlie, R. Zamoyska, T cell receptor signalling networks: branched, diversified and bounded. Nat Rev Immunol 13, 257-269 (2013).
26. J. H. Cho et al., CD45-mediated control of TCR tuning in naive and memory CD8(+) T cells. Nat Commun 7, 13373 (2016).
27. A. C. Maue et al., T-cell immunosenescence: lessons learned from mouse models of aging. Trends Immunol 30, 301-305 (2009).
28. J. Nikolich-Zugich, Aging of the T cell compartment in mice and humans: from no naive expectations to foggy memories. J Immunol 193, 2622-2629 (2014).
29. M. Yang, K. H. Vousden, Serine and one-carbon metabolism in cancer. Nat Rev Cancer 16, 650-662 (2016).
30. G. S. Ducker, J. D. Rabinowitz, One-Carbon Metabolism in Health and Disease. Cell Metab 25, 27-42 (2017).
31. N. Ron-Harel et al., Mitochondrial Biogenesis and Proteome Remodeling Promote One-Carbon Metabolism for T Cell Activation. Cell Metab 24, 104-117 (2016).
32. E. H. Ma et al., Serine Is an Essential Metabolite for Effector T Cell Expansion. Cell Metab 25, 345-357 (2017).
33. R. J. Morscher et al., Mitochondrial translation requires folate-dependent tRNA methylation. Nature 554, 128-132 (2018).
34. D. R. Minton et al., Serine Catabolism by SHMT2 Is Required for Proper Mitochondrial Translation Initiation and Maintenance of Formylmethionyl-tRNAs. Mol Cell 69, 610-621 e615 (2018).
35. N. Ron-Harel et al., Defective respiration and one-carbon metabolism contribute to impaired naive T cell activation in aged mice. Proc Natl Acad Sci U S A 115, 13347-13352 (2018).
36. C. H. June, Principles of adoptive T cell cancer therapy. J Clin Invest 117, 1204-1212 (2007).
37. N. S. Joshi, S. M. Kaech, Effector CD8 T cell development: a balancing act between memory cell potential and terminal differentiation. J Immunol 180, 1309-1315 (2008).
38. N. E. Scharping et al., The Tumor Microenvironment Represses T Cell Mitochondrial Biogenesis to Drive Intratumoral T Cell Metabolic Insufficiency and Dysfunction. Immunity 45, 701-703 (2016).
39. K. Chamoto et al., Mitochondrial activation chemicals synergize with surface receptor PD-1 blockade for T cell-dependent antitumor activity. Proc Natl Acad Sci U S A 114, E761-E770 (2017).
40. H. Wang et al., ZAP-70: an essential kinase in T-cell signaling. Cold Spring Harb Perspect Biol 2, a002279 (2010).
41. M. Perron, H. U. Saragovi, Inhibition of CD45 Phosphatase Activity Induces Cell Cycle Arrest and Apoptosis of CD45(+) Lymphoid Tumors Ex Vivo and In Vivo. Mol Pharmacol 93, 575-580 (2018).
42. M. Sharpe, N. Mount, Genetically modified T cells in cancer therapy: opportunities and challenges. Dis Model Mech 8, 337-350 (2015).
43. R. W. Jenkins, D. A. Barbie, K. T. Flaherty, Mechanisms of resistance to immune checkpoint inhibitors. Br J Cancer 118, 9-16 (2018).
44. R. J. DeBerardinis, N. S. Chandel, Fundamentals of cancer metabolism. Sci Adv 2, e1600200 (2016).
45. N. M. Chapman, M. R. Boothby, H. Chi, Metabolic coordination of T cell quiescence and activation. Nat Rev Immunol 20, 55-70 (2020).
46. E. S. Egorov et al., The Changing Landscape of Naive T Cell Receptor Repertoire With Human Aging. Front Immunol 9, 1618 (2018).
47. C. S. Palmer et al., Emerging Role and Characterization of Immunometabolism: Relevance to HIV Pathogenesis, Serious Non-AIDS Events, and a Cure. J Immunol 196, 4437-4444 (2016).
48. H. Tsukamoto, S. Senju, K. Matsumura, S. L. Swain, Y. Nishimura, IL-6-mediated environmental conditioning of defective Th1 differentiation dampens antitumour immune responses in old age. Nat Commun 6, 6702 (2015).
49. R. A. Miller, Effect of aging on T lymphocyte activation. Vaccine 18, 1654-1660 (2000).
50. G. Pawelec, K. Hirokawa, T. Fulop, Altered T cell signalling in ageing. Mech Ageing Dev 122, 1613-1637 (2001).
51. R. L. Whisler, M. Chen, B. Liu, Y. G. Newhouse, Age-related impairments in TCR/CD3 activation of ZAP-70 are associated with reduced tyrosine phosphorylations of zeta-chains and p59fyn/p561ck in human T cells. Mech Ageing Dev 111, 49-66 (1999).
52. T. Fulop, Jr., A. Larbi, G. Dupuis, G. Pawelec, Ageing, autoimmunity and arthritis: Perturbations of TCR signal transduction pathways with ageing - a biochemical paradigm for the ageing immune system. Arthritis Res Ther 5, 290-302 (2003).
53. T. Furukawa, M. Itoh, N. X. Krueger, M. Streuli, H. Saito, Specific interaction of the CD45 protein-tyrosine phosphatase with tyrosine-phosphorylated CD3 zeta chain. Proc Natl Acad Sci U S A 91, 10928-10932 (1994).
54. K. F. Lindahl, D. B. Wilson, Histocompatibility antigen-activated cytotoxic T lymphocytes. II. Estimates of the frequency and specificity of precursors. J Exp Med 145, 508-522 (1977).
55. M. Cascalho, J. L. Platt, Xenotransplantation and other means of organ replacement. Nat Rev Immunol 1, 154-160 (2001).
56. N. Degauque, S. Brouard, J. P. Soulillou, Cross-Reactivity of TCR Repertoire: Current Concepts, Challenges, and Implication for Allotransplantation. Front Immunol 7, 89 (2016).
57. L. J. D'Orsogna, D. L. Roelen, Doxiadis, II, F. H. Claas, TCR cross-reactivity and allorecognition: new insights into the immunogenetics of allorecognition. Immunogenetics 64, 77-85 (2012).
58. M. Hebeisen et al., Molecular insights for optimizing T cell receptor specificity against cancer. Front Immunol 4, 154 (2013).
59. H. Nishimura, N. Minato, T. Nakano, T. Honjo, Immunological studies on PD-1 deficient mice: implication of PD-1 as a negative regulator for B cell responses. Int Immunol 10, 1563-1572 (1998).
60. P. S. Chowdhury, K. Chamoto, A. Kumar, T. Honjo, PPAR-Induced Fatty Acid Oxidation in T Cells Increases the Number of Tumor-Reactive CD8(+) T Cells and Facilitates Anti-PD-1 Therapy. Cancer Immunol Res 6, 1375-1387 (2018).
61. M. D. Brand, D. G. Nicholls, Assessing mitochondrial dysfunction in cells. Biochem J 435, 297-312 (2011).

### [Example 2]

### [Materials and Methods]

### Mice and Cells

All mice were maintained under specific pathogen (that would hinder experiments)-free conditions at the Institute of Laboratory Animals, Graduate School of Medicine, Kyoto University, and used according to appropriate experimental guidelines. C57BL/6 mice were purchased from Charles River Laboratories Japan. PD-1^{-/-} (PD-1 KO) mice were prepared by homologous recombination in the laboratory of the present inventors (see Nishimura, et al.) (11). Murine colon adenocarcinoma-derived MC38 cells were kindly provided by J. P. Allison, Memorial Sloan-Kettering Cancer (New York, NY). Lewis lung carcinoma-derived LLC cells were purchased from American Type Culture Collection (ATCC). Cells were maintained in RPMI 1640 (Gibco; 11875-093) supplemented with 10% heat-inactivated fetal bovine serum (FBS) and 1% penicillin-streptomycin (Nacalai Tesque; 26253-84) under mycobacterial infection-free conditions.

### Mouse Therapy Models

CD45 inhibition (Fig. 12): 2-(4-acetylanilino)-3-chloronaphthoquinone (211) (530197, EDM Millipore) (12) (3 mg/kg) or N-(9,10-dioxo-9,10-dihydrophenanthren-2-yl)-2,2-dimethylpropionamide (PTP) (540215, Sigma-Aldrich) (13) (3 mg/kg) was administered intraperitoneally to mice before tumor cell inoculation or 5 or 7 days after tumor cell inoculation. MC38 cells (5 × 10⁵ or 1 × 10⁶) or LLC cells (2 × 10⁶) were intradermally inoculated into the right flank of mice (day 0). Seven days after LLC cell inoculation (day 7), mice received intraperitoneal administration of anti-PD-L1 antibody (clone 1-111A.4) (2 mg/kg). Administration of this anti-PD-L1 antibody was repeated 3 times at an interval of 6 days (on days 7, 13, and 19). Tumor size was measured with calipers, and tumor volume was calculated using the following formula: π (length × breadth × height)/6.

### Statistical Analysis

Data were analyzed using Prism 7 (Graph pad). When comparing more than two groups, statistical significance was analyzed using one-way analysis of variance (ANOVA) for multiple groups, followed by Tukey's post hoc test to analyze differences. Comparisons of two groups were analyzed using Student's t-test.

### Validation of Increase in Antitumor Effect caused by PD-1 Blockade with CD45 Inhibitor

In this Example, 2-(4-acetylanilino)-3-chloronaphthoquinone (211) or N-(9,10-dioxo-9,10-dihydrophenanthren-2-yl)-2,2-dimethylpropionamide (PTP) was used as CD45 inhibitor to examine the efficacy of tumor growth inhibition by combined use with PD-1 blockade (Fig. 12).

CD45 inhibitor (211) was administered intraperitoneally at 3 mg/kg to PD-1 KO mice (2-month old) once 3 days before inoculation of MC38 murine colon adenocarcinoma cells (preadministration) or 3 times 5 days after inoculation of MC38 cells (postadministration) (Fig. 13A and B). MC38 cells were intradermally inoculated into the right flank of miceat 1 × 10⁶ cells/tumor. As a result, tumor volume was significantly reduced in groups which received 211 administration before or after MC38 inoculation, compared with control group which received vehicle alone (Fig. 14A and B). These results suggest that CD45 inhibition enhances the antitumor effect caused by PD-1 blockade.

### Examination of CD45 Inhibitor's Improvement Effect on Aging-Related Resistance to PD-1 Blockade

CD45 inhibitor (211) was administered intraperitoneally at 3 mg/kg to young (1 to 2-month old) or aged (14 to 16-month old) PD-1 KO mice (2-month old). Three days after administration of 211, MC38 cells were intradermally inoculated into the right flank of mice at 5 × 10⁵ cells/tumor, followed by monitoring tumor growth. As a result, inhibition of tumor growth observed in young PD-1 KO mice was not observed in aged PD-1 KO mice, clearly revealing the aged mice's resistance to PD-1 blockade (Fig. 15A and B). On the other hand, when 211 was administered to aged PD-1 KO mice, tumor growth was strongly inhibited (Fig. 15A and B). These results indicate that CD45 inhibition suppresses the tumor growth representing age-related resistance to PD-1 blockade.

### Examination of CD45 Inhibitor's Improvement Effect on Cancers Showing Resistance to PD-1 Blockade

LLC cells (2 × 10⁶ cells/tumor) were i.d. inoculated into the right flank of young (1 to 2-month old) WT mice (Fig. 16A). After 7 days, therapy was started using anti-PD-L1 antibody alone or in combination with CD45 inhibitor (211 or PTP) to examine tumor growth (Fig. 16A). As a result, combination therapy with CD45 inhibitor and anti-PD-L1 antibody significantly inhibited tumor growth, though tumor inhibitory effect was little observed when anti-PD-L1 antibody was used alone (Fig. 16B and C). Either of these two CD45 inhibitors showed similar inhibitory effect against tumor growth when used in combination with anti-PD-L1 antibody (Fig. 16B and C). Further, a therapeutic effect of using CD45 inhibitor alone was examined under similar conditions. As a result, significant inhibitory effect was not shown against LLC tumor growth (Fig. 16D and E). These results indicated a possibility that CD45 inhibition suppresses progress of unresponsive cancers by enhancing the effect of PD-1 blockade therapy.

### [Example 3]

MC38 cancer cells (5 × 10⁵) were intradermally (i.d.) inoculated into young (6-week old) C57BL/6 mice. After 9, 16 and 23 days, anti-PD-L1 antibody (clone 1-111A.4) (1.5 mg/kg) was i.d. administered to mice. In addition to these administrations, mitomycin C-treated splenocytes from Balb/c (allogeneic) (5 × 10⁵ cells/mouse or 2 × 10⁶ cells/mouse) were intravenously injected into mice (A) once 6 days after tumor inoculation, (B) only once 9 days after tumor inoculation, or (C) three times in total 9, 16 and 23 days after tumor inoculation, followed by measurement of chronological changes in tumor size (Fig. 18). Points in each line graph are mean values from 5 to 6 mice, and error bars represent standard error. * means p-value less than 0.05, and n.s. means not significant (comparison was made by Turkey's test after one-way ANOVA). These results revealed that three-time administration of allogeneic cells produce higher effect of combined use with immune check point inhibitor, than one-time administration thereof.

### References

1. S. L. Topalian et al., Safety, activity, and immune correlates of anti-PD-1 antibody in cancer. N Engl J Med 366, 2443-2454 (2012).
2. A. Ribas et al., Association of Pembrolizumab With Tumor Response and Survival Among Patients With Advanced Melanoma. JAMA 315, 1600-1609 (2016).
3. M. Reck et al., Pembrolizumab versus Chemotherapy for PD-L1-Positive Non-Small-Cell Lung Cancer. N Engl J Med 375, 1823-1833 (2016).
4. P. S. Chowdhury, K. Chamoto, T. Honjo, Combination therapy strategies for improving PD-1 blockade efficacy: a new era in cancer immunotherapy. J Intern Med 283, 110-120 (2018).
5. W. Zou, J. D. Wolchok, L. Chen, PD-L1 (B7-H1) and PD-1 pathway blockade for cancer therapy: Mechanisms, response biomarkers, and combinations. Sci Transl Med 8, 328rv324 (2016).
6. A. L. Shergold, R. Millar, R. J. B. Nibbs, Understanding and overcoming the resistance of cancer to PD-1/PD-L1 blockade. Pharmacol Res 145, 104258 (2019).
7. K. Chamoto et al., Mitochondrial activation chemicals synergize with surface receptor PD-1 blockade for T cell-dependent antitumor activity. Proc Natl Acad Sci U S A 114, E761-E770 (2017).
8. J. Rossy, D. J. Williamson, K. Gaus, How does the kinase Lck phosphorylate the T cell receptor? Spatial organization as a regulatory mechanism. Front Immunol 3, 167 (2012).
9. R. J. Brownlie, R. Zamoyska, T cell receptor signalling networks: branched, diversified and bounded. Nat Rev Immunol 13, 257-269 (2013).
10. J. H. Cho et al., CD45-mediated control of TCR tuning in naive and memory CD8(+) T cells. Nat Commun 7, 13373 (2016).
11. H. Nishimura, N. Minato, T. Nakano, T. Honjo, Immunological studies on PD-1 deficient mice: implication of PD-1 as a negative regulator for B cell responses. Int Immunol 10, 1563-1572 (1998).
12. M. D. Perron et al., Allosteric noncompetitive small molecule selective inhibitors of CD45 tyrosine phosphatase suppress T-cell receptor signals and inflammation in vivo. Mol Pharmacol 85, 553-563 (2014).
13. B. Y Ma et al., The lectin Jacalin induces human B-lymphocyte apoptosis through glycosylation-dependent interaction with CD45. Immunology 127, 477-488 (2009).
14. S. M. Krummey et al., CD45RB Status of CD8(+) T Cell Memory Defines T Cell Receptor Affinity and Persistence. Cell Rep 30, 1282-1291 e1285 (2020).

All publications, patents and patent applications cited herein are incorporated herein by reference in their entirety.

### Industrial Applicability

The pharmaceutical composition of the present invention is applicable as an anticancer agent, an anti-infective agent or a combination thereof.

### Sequence Listing Free Text

### <SEQ ID NOS: 1 to 10>

These sequences show the nucleotide sequences of primers.

## Claims

1. A pharmaceutical composition which comprises a substance capable of enhancing T cell receptor (TCR) signaling and is administered before, after or simultaneously with the administration of a PD-1 signaling inhibitor.

2. The pharmaceutical composition of claim 1, wherein the substance capable of enhancing TCR signaling is a CD45 inhibitor and/or a cell.

3. The pharmaceutical composition of claim 2, wherein the CD45 inhibitor is at least one compound selected from the group consisting of 2-(4-acetylanilino)-3-chloronaphthoquinone, N-(9,10-dioxo-9,10-dihydrophenanthren-2-yl)-2,2-dimethylpropionamide, and analogs thereof.

4. The pharmaceutical composition of claim 2, wherein the cell is a xenogeneic cell, an allogeneic cell or a combination thereof.

5. The pharmaceutical composition of any one of claims 1 to 4, wherein the PD-1 signaling inhibitor is an antibody.

6. The pharmaceutical composition of claim 5, wherein the antibody is at least one antibody selected from the group consisting of anti-PD-1 antibody, anti-PD-L1 antibody and anti-PD-L2 antibody.

7. The pharmaceutical composition of any one of claims 1 to 6, which is used as an anti-cancer agent, an anti-infective agent or a combination thereof.

8. A drug which enhances PD-1 signaling inhibitory activity, comprising a CD45 inhibitor and/or a cell.

9. A TCR signaling enhancer comprising a CD45 inhibitor and/or a cell.

10. A method of preventing and/or treating cancer, infection or a combination thereof, comprising administering to a subject a pharmaceutically effective amount of a substance capable of enhancing T cell receptor (TCR) signaling before, after or simultaneously with the administration of a PD-1 signaling inhibitor.

11. A method of enhancing PD-1 signaling inhibitory activity, comprising administering to a subject a pharmaceutically effective amount of a substance capable of enhancing T cell receptor (TCR) signaling before, after or simultaneously with the administration of a PD-1 signaling inhibitor.

12. A method of enhancing TCR signaling, comprising administering to a subject a pharmaceutically effective amount of a CD45 inhibitor and/or a cell.

13. A substance capable of enhancing T cell receptor (TCR) signaling for use in prevention and/or treatment of cancer, infection or a combination thereof, said substance being administered before, after or simultaneously with the administration of a PD-1 signaling inhibitor.
